# EUROPEAN PATENT SPECIFICATION

(11) **EP 2 451 837 B1**
(45) Date of publication and mention of the grant of the patent: **25.03.2015**
(21) Application number: 10732589.6
(22) Date of filing: 07.07.2010
(51) Int. Cl.: C07K 14/715, C12N 5/071

(54) **MODIFIED IPS CELLS HAVING A MUTANT FORM OF HUMAN IMMUNODEFICIENCY VIRUS (HIV) CELLULAR ENTRY GENE**
MODIFIZIERTE IPS-ZELLEN MIT EINER MUTANTEN FORM DES HIV-ZELLEINTRITTSGENS
CELLULES IPS MODIFIÉES AYANT UNE FORME MUTANTE DU GÈNE D ENTRÉE CELLULAIRE DU VIRUS DE L`IMMUNODÉFICIENCE HUMAINE (VIH)

(30) Priority: 08.07.2009 US 223890 P
(43) Date of publication of application: 16.05.2012
(73) Proprietor: Cellular Dynamics International, Inc., Madison, WI 53711 (US)
(72) Inventor: THOMSON, James, Madison, WI 53711 (US); SEAY, Nick, Madison, WI 53711 (US)
(74) Representative: Vossius & Partner
(86) International application number: PCT/US2010/041194
(87) International publication number: WO 2011/005849

(56) References cited:
- WO-A1-2009/054985
- WO-A2-01/12857
- WO-A2-2009/152485
- AMABILE G ET AL: "Induced pluripotent stem cells: current progress and potential for regenerative medicine" TRENDS IN MOLECULAR MEDICINE, ELSEVIER CURRENT TRENDS, GB LNKD- DOI:10.1016/J.MOLMED.2008.12.003, vol. 15, no. 2, 1 February 2009 (2009-02-01), pages 59-68, XP025942032 ISSN: 1471-4914 [retrieved on 2009-01-21]
- YU JUNYING ET AL: "Human induced pluripotent stem cells free of vector and transgene sequences." SCIENCE (NEW YORK, N.Y.) 8 MAY 2009 LNKD- PUBMED:19325077, vol. 324, no. 5928, 8 May 2009 (2009-05-08), pages 797-801, XP002596377 ISSN: 1095-9203

## Description

This application claims priority to U.S. Application No. 61/223,890 filed on July 8, 2009.

The present invention relates generally to the field of molecular biology and cell biology. More particularly, it concerns compositions and methods relevant to modified induced pluripotent stem (iPS) cells and hematopoietic cells derived therefrom.

Human immunodeficiency virus (HIV) infection is most commonly treated with agents that interfere with viral replication, such as small molecule protease inhibitors, nucleoside analogues, and non-nucleoside reverse transcriptase inhibitors. These antiviral therapies have been relatively effective for reducing viral loads and restoring immune function. However, these drugs exhibit numerous side effects, require prolonged treatment that often induces drug resistance, and do not result in complete eradication of the virus from the body. As a consequence, a great deal of current research focuses on developing therapies which either enhance the ability of the immune system to neutralize HIV or interfere with the ability of the virus to infect immune cells. In particular, these therapies exploit the growing body of evidence that certain gene polymorphisms are associated with reduced susceptibility and disease progression (see, Roger *et al.,* 1998; O'Brien *et al.,* 1998; Hogan *et al.,* 2001).

The discovery that certain polymorphisms confer resistance to HIV has led to the proposal of therapies which repopulate the immune system with cells more capable of resisting infection and/or more capable of neutralizing the virus. By preventing *de novo* infection of cells, such therapy may eliminate the need for prolonged treatment with inhibitors of viral replication. Furthermore, the specific nature of such therapies should reduce side effects.

However, transducing circulating T lymphocytes with disease resistance polymorphisms is problematic, since these cells are so widely disseminated that is it is difficult to reach all target cells using current vector delivery systems. Furthermore, infusions of stem cells from donors, whether *in vitro* engineered or not, are preferably performed after matching of HLA phenotypes. Differences between the donor and the recipient can cause rejection of the transplant or even worse, the immune cells of the donor tissue may attack the tissues of the host (graft-versus-host disease). Current methods do not allow rapid and efficient production or identification of cells expressing both the desired disease resistance genes and HLA phenotype.

Thus there remains a need for cells and methods that may effectively render immune cells refractory to HIV infection and/or enhances the ability of the immune system to neutralize the virus with a reduced risk of immunologic incompatibility.

The present disclosure overcomes a major deficiency in the art by providing modified iPS cells related to human immunodeficiency virus (HIV) research, and to prevention and therapy for any diseases arising from HIV infection. For example, somatic cells may be obtained from a subject having or at risk of HIV infection and may be used to generate genetically modified iPS cells for resistance to HIV infection. This could obviate the common HLA type mismatching in transplantation therapy.
The invention relates to the embodiments as defined in the claims.

Thus, the invention relates to the following items.
1. An isolated induced pluripotent stem (iPS) cell for use in prevention and therapy of HIV infection, AIDS and/or ARC, wherein said cell has a modified gene structure comprising a mutant form of an HIV cellular entry gene selected from the group consisting of CCR5, CXCR4, CCR3, CCR2B, and CCR1, wherein said mutant form is a null genotype or encodes an inactive protein form.
2. The isolated iPS cell of item 1, wherein said gene structure is modified by homologous recombination or nuclease targeting.
3. The isolated iPS cell of item 1 or 2, wherein said CCR5 mutant is a 32 base-pair deletion in the coding region of wild-type CCR5 (CCR5 delta32) and/or wherein said CCR5 mutant is a CCR5m303 mutant.
4. The isolated iPS cell of any one of items 1 to 3, wherein said isolated iPS cell is homozygous for said CCR5 mutant.
5. An isolated modified hematopoietic cell differentiated from the isolated iPS cell of any one of items 1 to 4.
6. The isolated iPS cell of item 5, wherein said isolated hematopoietic cell is a hematopoietic stem cell, a hematopoietic progenitor cell, a T lymphocyte, a B lymphocyte, a mast cell, or a macrophage.
7. An *in vitro* method for making a modified iPS cell for use in prevention and therapy of HIV infection, AIDS and/or ARC, wherein said method comprises:
   a) obtaining a somatic cell, wherein said somatic cell is from a subject having or at risk of having an HIV infection or disorder;
   b) reprogramming said somatic cell to provide an induced pluripotent stem cell (iPS cell); and
   c) modifying said iPS cell to provide a modified iPS cell having a modified gene structure comprising a mutant form of an HIV cellular entry gene selected from the group consisting of CCR5, CXCR4, CCR3, CCR2B, and CCR1.
8. The *in vitro* method of item 7, further comprising: d) inducing differentiation of said modified iPS cell to provide a modified hematopoietic cell.
9. The *in vitro* method of item 7 or 8, wherein said modifying comprises homologous recombination or nuclease targeting.
10. The *in vitro* method of any one of items 7 to 9, wherein said modified gene structure is introduced into said iPS cell by a vector.
11. The *in vitro* method of any one of items 7 to 10, wherein said modified gene structure comprises a CCR5 null genotype or a CCR5 mutant encoding an inactive form of CCR5.
12. The *in vitro* method of item 11, wherein said modifying comprises replacing one or two endogenous CCR5 alleles with said CCR5 mutant.
13. The *in vitro* method of item 11 or 12, wherein said CCR5 mutant is a 32 base-pair deletion in the coding region of wild-type CCR5 (CCR5 delta32) and/or wherein said CCR5 mutant is a CCR5m303 mutant.
14. The *in vitro* method of any one of items 11 to 13, wherein said modified iPS cell is homozygous for said CCR5 mutants.
15. The *in vitro* method of any one of items 7 to 14, wherein said subject is human.

In a first embodiment, there may be provided an isolated induced pluripotent stem (iPS) cell for use in prevention and therapy of HIV infection, AIDS and/or ARC, wherein said cell has a modified gene structure comprising a mutant form of an HIV cellular entry gene selected from the group consisting of CCR5, CXCR4, CCR3, CCR2B, and CCR1, wherein the mutant form is a null genotype or encodes an inactive protein form. The gene structure may be modified by introduction of exogenous genetic material, such as homologous recombination, nuclease targeting, or any genetic engineering method known in the art.

In certain aspects, the modified gene structure may comprise a CCR5 null genotype or a CCR5 mutant encoding an inactive form of CCR5 protein. The CCR5 mutant may be a mutant form of a wild-type CCR5 gene. The human wild-type CCR5 gene is any native variant that encodes wild-type CCR protein (Genbank accession number NP_000570.1), such as wild-type genes designated as Genbank accession number NM_000579 or NM_001100168.1.

For example, such a CCR5 mutant may comprise a 32 base-pair deletion in the coding region of human wild-type CCR5 (CCR5 delta32). The 32-base-pair deletion within the coding region (CCR5 delta32) is deposited in the Genbank database, assigned accession No. X99393, which has a deletion of nucleic acid residues 793-824 of the wild-type sequence. CCR5 delta32 results in a frame shift, and generates a non-functional receptor that does not support membrane fusion or infection by macrophage- and dual-tropic HIV-1 strains (Samson et al., 1996).

The CCR5 mutant may also comprise a CCR5m303 mutant. CCR5m303 mutant encodes a mutant protein that has a Thr → Ala CCR5 mutation at nucleotide position 303 of wild-type CCR5 protein, which causes an in-frame stop at the distal end of the first extracellular loop of CCR5 and abolishes coreceptor activity for its cognate gene product (see U.S. Patent No. 6,153,431).

The modified gene structure may also comprise a mutant form of a wild-type CXCR4, CCR3, CCR2B, and/or CCR1. The wild-type human CXCR4 (chemokine (C-X-C motif) receptor 4) gene, designated as AJ224869 in Genbank database, encodes a wild-type protein designated as CAA12166 in Genbank database. The wild-type human CCR3 (chemokine (C-C motif) receptor 3) gene, including isoforms NM_178328.1 and NM_001837.3 as designated in Genbank database, encodes a wild-type protein designated as NP_847899.1 in Genbank database. The wild-type CCR2B (C-C chemokine receptor type 2 isoform B), designated as NM_001123396.1 in Genbank database, encodes a wild-type protein designated as NP_001116868.1 in Genbank database. The wild-type human CCR1 (chemokine (C-C motif) receptor 1) gene, designated as NM_001295.2 in Genbank database, encodes a wild-type protein designated as NP_001286.1 in Genbank database.

The isolated iPS cell may be homozygous for the CCR5 mutant. In a certain embodiment, there may be provided an isolated hematopoietic cell differentiated from the isolated iPS cell described above. Examples of the isolated modified hematopoietic cell include, but are not limited to, a hematopoietic stem cell, a hematopoietic progenitor cell, a T lymphocyte, a B lymphocyte, a mast cell, or a macrophage. In certain aspects, the iPS cell is a human iPS cell, more particularly, an iPS cells derived from a human at risk of or having an HIV infection or disorder.

In certain further embodiments, the invention involves an *in vitro* method for making a modified iPS cell, for use in prevention and therapy of HIV infection, AIDS and/or ARC, wherein said method comprises: a) obtaining a somatic cell, wherein the somatic cell is from a subject having or at risk of having an HIV infection or disorder; b) reprogramming the somatic cell to provide.an induced pluripotent stem cell (iPS cell); and c) modifying the iPS cell to provide a modified iPS cell having a modified gene structure comprising a mutant form of an HIV cellular entry gene selected from the group consisting of CCR5, CXCR4, CCR3, CCR2B, and CCR1. The method may further comprise d) inducing differentiation of the modified iPS cell to provide a modified hematopoietic cell.

In certain aspects of the method, the gene structure may be modified by homologous recombination, nuclease targeting, or any genetic engineering method known in the art. For example, the modified gene structure may comprise a CCR5 null genotype or a CCR5 mutant encoding an inactive form of CCR5 protein, for example, such a CCR5 mutant may be a CCR5 delta32 or a CCR5m303 mutant, or a combination thereof. The modified gene structure may be introduced into the iPS cell by a vector, such as a gene targeting vector. The modifying method may comprise replacing one or two endogenous CCR5 alleles with the CCR5 mutant. In a further embodiment, the modified iPS cell may be homozygous for the CCR5 mutant. Furthermore, in highly preferred aspects of the invention, the subject may be a mammal, such as a human.

Embodiments discussed in the context of methods and/or compositions of the disclosure may be employed with respect to any other method or composition described herein. Thus, an embodiment pertaining to one method or composition may be applied to other methods and compositions of the disclosure as well.

As used herein the terms "encode" or "encoding" with reference to a nucleic acid are used to make the invention readily understandable by the skilled artisan; however, these terms may be used interchangeably with "comprise" or "comprising", respectively.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only.

The present disclosure, according to certain embodiments, is generally directed to induced pluripotent stem cells or hematopoietic cells having modified gene structure relevant to HIV cellular entry and methods of providing such cells.

"Modified gene structure" is used herein to mean a gene structure altered genetically from a wild-type gene structure, preferably through artificial means other than natural selection.

The term "wild-type" refers to the typical form of a gene, a gene product, or a characteristic of that gene or gene product when isolated from a naturally occurring source. A wild-type is that which is most frequently observed in a nature population and is thus arbitrarily designated the native, normal or wild-type form. In contrast, the term "modified," "variant," or "mutant" refers to a gene or gene product which displays modification in sequence and functional properties (i.e., altered characteristics) when compared to the wild-type gene or gene product. It is noted that naturally-occurring mutants can be isolated; they are identified by the fact that they have altered characteristics when compared to the wild-type gene or gene product.

"Reprogramming" is a process that confers on a cell a measurably increased capacity to form progeny of at least one new cell type, either in culture or *in vivo*, than it would have under the same conditions without reprogramming. More specifically, reprogramming is a process that confers on a somatic cell a pluripotent potential. This means that after sufficient proliferation, a measurable proportion of progeny having phenotypic characteristics of the new cell type if essentially no such progeny could form before reprogramming; otherwise, the proportion having characteristics of the new cell type is measurably more than before reprogramming. Under certain conditions, the proportion of progeny with characteristics of the new cell type may be at least about 1%, 5%, 25% or more in the in order of increasing preference.

A "vector" or "construct" (sometimes referred to as gene delivery or gene transfer "vehicle") refers to a macromolecule or complex of molecules comprising a polynucleotide to be delivered to a host cell, either *in vitro* or *in vivo.* A vector can be a linear or a circular molecule.

A "plasmid", a common type of a vector, is an extra-chromosomal DNA molecule separate from the chromosomal DNA which is capable of replicating independently of the chromosomal DNA. In certain cases, it is circular and double-stranded.

By "expression construct" or "expression cassette" is meant a nucleic acid molecule that is capable of directing transcription. An expression construct includes, at the least, a promoter or a structure functionally equivalent to a promoter. Additional elements, such as an enhancer, and/or a transcription termination signal, may also be included.

The term "exogenous," when used in relation to a protein, gene, nucleic acid, or polynucleotide in a cell or organism refers to a protein, gene, nucleic acid, or polynucleotide which has been introduced into the cell or organism by artificial or natural means, or in relation a cell refers to a cell which was isolated and subsequently introduced to other cells or to an organism by artificial or natural means. An exogenous nucleic acid may be from a different organism or cell, or it may be one or more additional copies of a nucleic acid which occurs naturally within the organism or cell. An exogenous cell may be from a different organism, or it may be from the same organism. By way of a non-limiting example, an exogenous nucleic acid is in a chromosomal location different from that of natural cells, or is otherwise flanked by a different nucleic acid sequence than that found in nature.

The term "corresponds to" is used herein to mean that a polynucleotide sequence is homologous (*i.e.,* is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

A "gene," "polynucleotide," "coding region," "sequence, " "segment," "fragment," or "transgene" which "encodes" a particular protein, is a nucleic acid molecule which is transcribed and optionally also translated into a gene product, e.g., a polypeptide, *in vitro* or *in vivo* when placed under the control of appropriate regulatory sequences. The coding region may be present in either a cDNA, genomic DNA, or RNA form. When present in a DNA form, the nucleic acid molecule may be single-stranded (*i.e.,* the sense strand) or double-stranded. The boundaries of a coding region are determined by a start codon at the 5' (amino) terminus and a translation stop codon at the 3' (carboxy) terminus. A gene can include, but is not limited to, cDNA from prokaryotic or eukaryotic mRNA, genomic DNA sequences from prokaryotic or eukaryotic DNA, and synthetic DNA sequences. A transcription termination sequence will usually be located 3' to the gene sequence.

The term "cell" is herein used in its broadest sense in the art and refers to a living body which is a structural unit of tissue of a multicellular organism, is surrounded by a membrane structure which isolates it from the outside, has the capability of self replicating, and has genetic information and a mechanism for expressing it. Cells used herein may be naturally-occurring cells or artificially modified cells (*e.g.,* fusion cells, genetically modified cells, *etc.*).

As used herein, the term "stem cell" refers to a cell capable of self replication and pluripotency. Typically, stem cells can regenerate an injured tissue. Stem cells herein may be, but are not limited to, induced pluripotent stem cells, embryonic stem (ES) cells or tissue stem cells (also called tissue-specific stem cell, or somatic stem cell). Any artificially produced cell which can have the above-described abilities (*e.g.,* fusion cells, reprogrammed cells, or the like used herein) may be a stem cell.

"Embryonic stem (ES) cells" are pluripotent stem cells derived from early embryos. An ES cell was first established in 1981, which has also been applied to production of knockout mice since 1989. In 1998, a human ES cell was established, which is currently becoming available for regenerative medicine.

Unlike ES cells, tissue stem cells have a limited differentiation potential. Tissue stem cells are present at particular locations in tissues and have an undifferentiated intracellular structure. Therefore, the pluripotency of tissue stem cells is typically low. Tissue stem cells have a higher nucleus/cytoplasm ratio and have few intracellular organelles. Most tissue stem cells have low pluripotency, a long cell cycle, and proliferative ability beyond the life of the individual. Tissue stem cells are separated into categories, based on the sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, liver stem cells, and the like. Tissue stem cells in the bone marrow system include hematopoietic stem cells, mesenchymal stem cells, and the like. Tissue stem cells in the nervous system include neural stem cells, retinal stem cells, and the like.

"Induced pluripotent stem cells," commonly abbreviated as iPS cells or iPSCs, refer to a type of pluripotent stem cell artificially prepared from a non-pluripotent cell, typically an adult somatic cell, or terminally differentiated cell, such as fibroblast, a hematopoietic cell, a myocyte, a neuron, an epidermal cell, or the like, by introducing certain factors, referred to as reprogramming factors.

"Pluripotency" refers to a stem cell that has the potential to differentiate into all cells constituting one or more tissues or organs, or preferably, any of the three germ layers: endoderm (interior stomach lining, gastrointestinal tract, the lungs), mesoderm (muscle, bone, blood, urogenital), or ectoderm (epidermal tissues and nervous system). "Pluripotent stem cells" used herein refer to cells that can differentiate into cells derived from any of the three germ layers, for example, direct descendants of totipotent cells or induced pluripotent cells.

By "operably linked" with reference to nucleic acid molecules is meant that two or more nucleic acid molecules (*e.g.,* a nucleic acid molecule to be transcribed, a promoter, and an enhancer element) are connected in such a way as to permit transcription of the nucleic acid molecule. "Operably linked" with reference to peptide and/or polypeptide molecules is meant that two or more peptide and/or polypeptide molecules are connected in such a way as to yield a single polypeptide chain, *i.e.,* a fusion polypeptide, having at least one property of each peptide and/or polypeptide component of the fusion. The fusion polypeptide is preferably chimeric, *i.e.,* composed of heterologous molecules.

"Homology" refers to the percent of identity between two polynucleotides or two polypeptides. The correspondence between one sequence and to another can be determined by techniques known in the art. For example, homology can be determined by a direct comparison of the sequence information between two polypeptide molecules by aligning the sequence information and using readily available computer programs. Alternatively, homology can be determined by hybridization of polynucleotides under conditions which form stable duplexes between homologous regions, followed by digestion with single strand-specific nuclease(s), and size determination of the digested fragments. Two DNA, or two polypeptide, sequences are "substantially homologous" to each other when at least about 80%, preferably at least about 90%, and most preferably at least about 95% of the nucleotides, or amino acids, respectively match over a defined length of the molecules, as determined using the methods above.

### Human Immunodeficiency Virus (HIV)

Drug resistance in treatment of HIV infection is becoming increasingly widespread and problematic. Resistance to one inhibitor targeting the viral enzymes reverse transcriptase (RT) or protease can often confer cross-resistance to other inhibitors within the same class and some individuals carry viruses resistant to a number of different drugs. These drug resistant viruses can be transmitted, leaving some newly infected as well as drug experienced individuals with little options for effective therapy. This highlights the importance of the development of new antiretroviral agents with novel mechanisms of action that will be active against viruses resistant to current therapeutics.

In certain aspects of the present invention, novel induced pluripotent stem cells with modified gene structure in HIV cellular entry genes have been disclosed. Those cells may be used to generate hematopoietic cells for treating HTV infection.

### A. HIV Infection

Human immunodeficiency virus (HIV) is a lentivirus (a member of the retrovirus family) that can lead to *acquired immunodeficiency syndrome* (AIDS), a condition in humans in which the immune system begins to fail, leading to life-threatening opportunistic infections. Previous names for the virus include human T-lymphotropic virus-III (HTLV-III), lymphadenopathy-associated virus (LAV), and AIDS-associated retrovirus (ARV) (Sowadsky, 2009; Coffin *et al.,* 1986).

Infection with HIV occurs by the transfer of blood, semen, vaginal fluid, pre-ejaculate, or breast milk. Within these bodily fluids, HIV is present as both free virus particles and virus within infected immune cells. The four major routes of transmission are unprotected sexual intercourse, contaminated needles, breast milk, and transmission from an infected mother to her baby at birth (Vertical transmission). Screening of blood products for HIV has largely eliminated transmission through blood transfusions or infected blood products in the developed world.

HIV infection in humans is now pandemic. As of January 2006, the Joint United Nations Programme on HIV/AIDS (UNAIDS) and the World Health Organization (WHO) estimate that AIDS has killed more than 25 million people since it was first recognized on December 1, 1981. It is estimated that about 0.6 percent of the world's population is infected with HIV (Joint United Nations Programme on HIV/AIDS, 2006). In 2005 alone, AIDS claimed an estimated 2.4-3.3 million lives, of which more than 570,000 were children. A third of these deaths are occurring in sub-Saharan Africa, retarding economic growth and increasing poverty (Greener, 2002). According to current estimates, HIV is set to infect 90 million people in Africa, resulting in a minimum estimate of 18 million orphans (Joint United Nations Programme on HIV/AIDS, 2005). Antiretroviral treatment reduces both the mortality and the morbidity of HIV infection, but routine access to antiretroviral medication is not available in all countries (Palella *et al.,* 1998).

HIV primarily infects vital cells in the human immune system such as helper T cells (specifically CD4⁺ T cells), macrophages, and dendritic cells. HIV infection leads to low levels of CD4⁺ T cells through three main mechanisms: firstly, direct viral killing of infected cells; secondly, increased rates of apoptosis in infected cells; and thirdly, killing of infected CD4⁺ T cells by CD8 cytotoxic lymphocytes that recognize infected cells. When CD4⁺ T cell numbers decline below a critical level, cell-mediated immunity is lost, and the body becomes progressively more susceptible to opportunistic infections.

Eventually most HIV-infected individuals develop AIDS. These individuals mostly die from opportunistic infections or malignancies associated with the progressive failure of the immune system (Lawn, 2004). Without treatment, about 9 out of every 10 persons with HIV will progress to AIDS after 10-15 years. Many progress much sooner (Buchbinder *et al.,* 1994). Treatment with anti-retrovirals increases the life expectancy of people infected with HIV. Even after HIV has progressed to diagnosable AIDS, the average survival time with antiretroviral therapy (as of 2005) is estimated to be more than 5 years (Schneider *et al.,* 2005). Without antiretroviral therapy, death normally occurs within a year (Morgan *et al.,* 2002).

### B. HIV Tropism

The term viral tropism refers to which cell types HIV infects. HIV can infect a variety of immune cells such as CD4⁺ T cells, macrophages, and microglial cells. HIV-1 entry to macrophages and CD4⁺ T cells is mediated through interaction of the virion envelope glycoproteins (gp120) with the CD4 molecule on the target cells and also with chemokine coreceptors (Chan *et al.,* 1997).

Macrophage (M-tropic) strains of HIV-1, or non-syncitia-inducing strains (NSI) use the β-chemokine receptor CCR5 for entry and are thus able to replicate in macrophages and CD4⁺ T cells (Coakley *et al.,* 2005). This CCR5 coreceptor is used by almost all primary HIV-1 isolates regardless of viral genetic subtype. Indeed, macrophages play a key role in several critical aspects of HIV infection. They appear to be the first cells infected by HIV and perhaps the source of HIV production when CD4⁺ cells become depleted in the patient. Macrophages and microglial cells are the cells infected by HIV in the central nervous system. In tonsils and adenoids of HIV-infected patients, macrophages fuse into multinucleated giant cells that produce huge amounts of virus.

T-tropic isolates, or syncitia-inducing (SI) strains replicate in primary CD4⁺ T cells as well as in macrophages and use the α-chemokine receptor, CXCR4, for entry (Coakley *et al.,* 2005; Deng *et al.,* 1996; Feng *et al.,* 1996). Dual-tropic HIV-1 strains are thought to be transitional strains of the HIV-1 virus and thus are able to use both CCR5 and CXCR4 as co-receptors for viral entry.

The α-chemokine SDF-1, a ligand for CXCR4, suppresses replication of T-tropic HIV-1 isolates. It does this by down-regulating the expression of CXCR4 on the surface of these cells. HIV that use only the CCR5 receptor are termed R5; those that only use CXCR4 are termed X4, and those that use both, X4R5. However, the use of coreceptor alone does not explain viral tropism, as not all R5 viruses are able to use CCR5 on macrophages for a productive infection (Coakley *et al.,* 2005) and HIV can also infect a subtype of myeloid dendritic cells (Knight *et al.,* 1990), which probably constitute a reservoir that maintains infection when CD4⁺ T cell numbers have declined to extremely low levels.

Some people are resistant to certain strains of HIV (Tang and Kaslow, 2003). One example of how this occurs is people with the CCR5-Δ32 mutation; these people are resistant to infection with R5 virus as the mutation stops HIV from binding to this coreceptor, reducing its ability to infect target cells.

Sexual intercourse is the major mode of HIV transmission. Both X4 and R5 HIV are present in the seminal fluid which is passed from a male to his sexual partner. The virions can then infect numerous cellular targets and disseminate into the whole organism. However, a selection process leads to a predominant transmission of the R5 virus through this pathway (Zhu *et al.,* 1993; van't Wout *et al.,* 1994; Zhu *et al.,* 1996). How this selective process works is still under investigation, but one model is that spermatozoa may selectively carry R5 HIV as they possess both CCR3 and CCR5 but not CXCR4 on their surface (Muciaccia *et al.,* 2005) and that genital epithelial cells preferentially sequester X4 virus (Berlier *et al.,* 2005). In patients infected with subtype B HIV-1, there is often a co-receptor switch in late-stage disease and T-tropic variants appear that can infect a variety of T cells through CXCR4 (Clevestig *et al.,* 2005). These variants then replicate more aggressively with heightened virulence that causes rapid T cell depletion, immune system collapse, and opportunistic infections that mark the advent of AIDS (Moore, 1997). Thus, during the course of infection, viral adaptation to the use of CXCR4 instead of CCR5 may be a key step in the progression to AIDS. A number of studies with subtype B-infected individuals have determined that between 40 and 50% of AIDS patients can harbour viruses of the SI, and presumably the X4, phenotype (Karlsson *et al.,* 1994; Koot *et al.,* 1996).

### C. HIV Cellular Entry

Gene structure relevant to HIV cellular entry may be modified in iPS cells for HIV infection study and treatment in certain aspects of the invention.

HIV enters macrophages and CD4⁺ T cells by the adsorption of glycoproteins on its surface to receptors on the target cell followed by fusion of the viral envelope with the cell membrane and the release of the HIV capsid into the cell (Chan and Kim, 1998; Wyatt and Sodroski, 1998).

Entry to the cell begins through interaction of the trimeric envelope complex (gp160 spike) and both CD4 and a chemokine receptor (generally either CCR5 or CXCR4, but others are known to interact) on the cell surface (Chan and Kim, 1998; Wyatt and Sodroski, 1998). gp120 binds to integrin α₄β₇ activating LFA-1 the central integrin involved in the establishment of virological synapses, which facilitate efficient cell-to-cell spreading of HIV-1 (Arthos *et al.,* 2008). The gp160 spike contains binding domains for both CD4 and chemokine receptors (Chan and Kim, 1998; Wyatt and Sodroski, 1998). The first step in fusion involves the high-affinity attachment of the CD4 binding domains of gp120 to CD4. Once gp120 is bound with the CD4 protein, the envelope complex undergoes a structural change, exposing the chemokine binding domains of gp120 and allowing them to interact with the target chemokine receptor (Chan and Kim, 1998; Wyatt and Sodroski, 1998). This allows for a more stable two-pronged attachment, which allows the N-terminal fusion peptide gp41 to penetrate the cell membrane (Chan and Kim, 1998; Wyatt and Sodroski, 1998). Repeat sequences in gp41, HR1 and HR2 then interact, causing the collapse of the extracellular portion of gp41 into a hairpin. This loop structure brings the virus and cell membranes close together, allowing fusion of the membranes and subsequent entry of the viral capsid (Chan and Kim, 1998; Wyatt and Sodroski, 1998).

After HIV has bound to the target cell, the HIV RNA and various enzymes, including reverse transcriptase, integrase, ribonuclease and protease, are injected into the cell(Chan and Kim, 1998). During the microtubule based transport to the nucleus, the viral single strand RNA genome is transcribed into double strand DNA, which is then integrated into a host chromosome.

HIV can infect dendritic cells (DCs) by this CD4-CCR5 route, but another route using mannose-specific C-type lectin receptors such as DC-SIGN can also be used (Pope and Haase, 2003). DCs are one of the first cells encountered by the virus during sexual transmission. They are currently thought to play an important role by transmitting HIV to T-cells when the virus is captured in the mucosa by DCs (Pope and Haase, 2003).

### HIV-related Polymorphisms

In certain aspects of the invention, genetically modified iPS cells may be provided, which may have a beneficial polymorphism, for example, relevant to reduction or prevention of HIV cellular entry. The term "beneficial" as described herein refers to any gene or gene structure which provides increased resistance to a disease of interest, reduces the progression of the disease, or is beneficial to research in disease resistance and/or progression.

Many of these beneficial polymorphisms are variants of receptors and ligands for receptors that mediate HIV entry into immune cells. Although human immunodeficiency virus type-1 ("HIV-1") uses the T cell surface molecule CD4 as a primary receptor, successful viral entry into and infection of a cell has been found to require the presence of a second molecule, or "co-receptor" (see, Clapham and Weiss, 1997). Seven co-receptor molecules have been identified, each of which are members of, or related to, the family of chemokine receptors, which are G-protein coupled receptors having seven transmembrane domains. The chemokine receptor CCR5, which selectively binds RANTES, MIP-1alpha, and MIP-1beta, serves as a coreceptor for macrophage tropic-strains of HIV, whereas the stromal derived factor 1 (SDF-1) chemokine receptor CXCR4 is a coreceptor for T cell-tropic HIV strains. CCR3, CCR2b, and CCR1 serve as coreceptors for other less common HIV strains.

The first HIV resistance gene to be characterized was a polymorphism of the primary HIV coreceptor CCR5 (see, Dean *et al.,* 1996; Liu *et al.,* 1996). CCR5, short for chemokine (C-C motif) receptor 5 is a protein which in humans is encoded by the CCR5 gene which is located on chromosome 3 on the short (p) arm at position 21. CCR5 has also recently been designated CD195 (cluster of differentiation 195).

HIV uses CCR5 or another protein, CXCR4, as a co-receptor to enter its target cells. Several chemokine receptors can function as viral coreceptors, but CCR5 is likely the most physiologically important coreceptor during natural infection. The normal ligands for this receptor, RANTES, MIP-1β, and MIP-1α, are able to suppress HIV-1 infection *in vitro.* In individuals infected with HIV, CCR5-using viruses are the predominant species isolated during the early stages of viral infection, suggesting that these viruses may have a selective advantage during transmission or the acute phase of disease. Moreover, at least half of all infected individuals harbor only CCR5-using viruses throughout the course of infection.

The CCR5 protein functions as a chemokine receptor in the CC chemokine group. The natural chemokine ligands that bind to this receptor are RANTES, MIP-1α and MIP-1β. CCR5 is predominantly expressed on T cells, macrophages, dendritic cells and microglia. It is likely that CCR5 plays a role in inflammatory responses to infection, though its exact role in normal immune function is unclear.

A 32 basepair deletion of the CCR5 receptor (CCR5-Δ32, or CCR5-D32 or CCR5 delta 32) causes a frameshift mutation and deletion of the last three transmembrane domains. While CCR5 has multiple variants in its coding region, the deletion of a 32-bp segment results in a nonfunctional receptor, thus preventing HIV R5 entry; two copies of this allele provide strong protection against HIV infection. This allele is found in 5-14% of Europeans but is rare in Africans and Asians. Multiple studies of HIV-infected persons have shown that presence of one copy of this allele delays progression to the condition of AIDS by about 2 years. Individuals homozygous for such a deletion remain uninfected despite multiple sexual exposures to HIV. CCR5-Δ32 decreases the number of CCR5 proteins on the outside of the CD4 cell, which can have a large effect on the HIV disease progression rates. It is possible that a person with the CCR5-Δ32 receptor allele will not be infected with HIV R5 strains. Several commercial testing companies offer tests for CCR5-Δ32.

In 2008, German doctors announced that an HIV-infected leukemia patient had received a bone marrow transplant from a donor who is homozygous for the CCR5-Δ32 trait (Hütter *et al.,* 2009). After 600 days, the patient was healthy and had undetectable levels of HIV in the blood and in examined brain and rectal tissues. Before the transplant, low levels of HIV X4, which does not use the CCR5 receptor, were also detected. Following the transplant, however, this type of HIV was not detected either, suggesting the central role of CCR5 in maintaining HIV-1 infection as the patient had non-CCR5-tropic X4 variants.

Another beneficial polymorphism is a point mutation at residue 303 of the CCR5 (CCR5 m303), which creates a stop codon and deletion of the last five transmembrane domains and the cytoplasmic tail. This mutation confers resistance to HIV infection when associated with the CCR5 delta 32 mutation (see, Quillent *et al.,* 1998; U.S. Pat. 6,153,431). A single amino acid substitution of CCR5, R89C also appears to confer resistance to HIV infection. (See, Tamasauskas *et al.,* 2001).

Polymorphisms (*e.g.,* CCR5P1, CCR5 59029A and 59353C) in the promoter region of these coreceptors may be associated with progression of the disease (see, Ometto *et al.,* 2001; Martin *et al..* 1998; Clegg *et al.,* 2000).

Variants of other less utilized HIV coreceptors also appear to influence disease progression. A conservative point mutation of the CCR2 receptor (CCR2-64L) permits expression of the receptor but nevertheless delays disease progression (see, Smith *et al.,* 1997).

Polymorphisms of genes encoding ligands for the HIV coreceptors CCR5 and CXCR4 influence disease progression, but not susceptibility. For example, homozygosity for a point mutation in the 3' untranslated region of a Stromal-derived Factor 1 alpha (SDF-1 alpha) delays disease progression in a recessive manner (see, Winkler *et al.,* 1998). It is hypothesized that the 3'A mutation upregulates the biosynthesis of SDF-1 alpha such that there is increased competition with HIV for CXCR4 receptors. A RANTES promoter polymorphism that increases RANTES expression is believed to function in a similar manner, but in this case by increasing competition with HIV for the CCR5 receptor (see, Liu *et al.,* 1999).

Finally, there is also evidence that HLA alleles influence HIV-1 disease progression. Animal studies demonstrate that resistance to murine AIDS maps to the H-2 complex, the mouse homologue of the HLA locus (see, Makino *et al.,* 1990). The HLA complex contains three types of genes (class I, II, and III), all of which are involved in modulating the immune response. Class I (A, B, C, D, E, F, G) and class II (DM, DP, DQ, DR) molecules, commonly known as MHC genes, are both involved in antigen presentation to T cells. Class m HLA includes a variety of unrelated proteins, including the transporter for antigen processing (TAP), polypeptides of the proteasome, complement component factors (Bf, C2, C4), and tumor necrosis factors (TNF-alpha, TNF-beta).

Studies indicate that an individual's particular type of MHC class I and II molecules can influence disease progression. A study of pairs of HIV-1 infected hemophiliac brothers has demonstrated that sibling pairs sharing one or two HLA class II alleles exhibit similar rates of disease progression (see, Kroner *et al.,* 1995). A more recent study has found that HLA class I B*5701 is highly associated with restriction of viral replication in nonprogressors (see, Flores-Villanueva *et al.,* 2001). It is hypothesized that an enhanced ability of certain MHC proteins to associate with processed HIV-1 antigens allows certain individuals to mount a highly effective CD8 lymphocyte response against the virus.

Another polymorphism that influences HIV disease progression is IL10-5'A, a variant of the promoter region for interleukin-10 (IL-10). This polymorphism reduces IL10 production and is associated with rapid progression of AIDS in both homozygotes and heterozygotes (see, Shin *et al.,* 2000). IL-10 is known to inhibit macrophage, T-lymphocyte, and HIV replication. Presumably, promoter mutations which increase IL-10 levels would slow progression of AIDS.

### Stem Cells

In certain embodiments of the disclosure, there are disclosed methods of using induced pluripotent stem cells (iPS cells) with modified gene structure. Those iPS cells may be made by reprogramming somatic cells and could be identical to embryonic stem cells in various aspects as described below. Understanding of embryonic stem cell characteristics could help select induced pluripotent stem cells. Reprogramming factors known from stem cell reprogramming studies could be used for these novel methods. It is further contemplated that these induced pluripotent stem cells could be potentially used to replace embryonic stem cells for therapeutics and research applications due to the ethics hurdle to use the latter.

### A. Stem Cells

Stem cells are cells found in most, if not all, multi-cellular organisms. They are characterized by the ability to renew themselves through mitotic cell division and differentiating into a diverse range of specialized cell types. The two broad types of mammalian stem cells are: embryonic stem cells that are found in blastocysts, and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin or intestinal tissues.

As stem cells can be grown and transformed into specialized cells with characteristics consistent with cells of various tissues such as muscles or nerves through cell culture, their use in medical therapies has been proposed. In particular, embryonic cell lines, autologous embryonic stem cells generated through therapeutic cloning, and highly plastic adult stem cells from the umbilical cord blood or bone marrow are touted as promising candidates. Most recently, the reprogramming of adult cells into induced pluripotent stem cells has enormous potential for replacing embryonic stem cells.

### B. Embryonic Stem Cells

Embryonic stem cell lines (ES cell lines) are cultures of cells derived from the epiblast tissue of the inner cell mass (ICM) of a blastocyst or earlier morula stage embryos. A blastocyst is an early stage embryo-approximately four to five days old in humans and consisting of 50-150 cells. ES cells are pluripotent and give rise during development to all derivatives of the three primary germ layers: ectoderm, endoderm and mesoderm. In other words, they can develop into each of the more than 200 cell types of the adult body when given sufficient and necessary stimulation for a specific cell type. They do not contribute to the extra-embryonic membranes or the placenta.

Nearly all research to date has taken place using mouse embryonic stem cells (mES) or human embryonic stem cells (hES). Both have the essential stem cell characteristics, yet they require very different environments in order to maintain an undifferentiated state. Mouse ES cells may be grown on a layer of gelatin and require the presence of Leukemia Inhibitory Factor (LIF). Human ES cells could be grown on a feeder layer of mouse embryonic fibroblasts (MEFs) and often require the presence of basic Fibroblast Growth Factor (bFGF or FGF-2). Without optimal culture conditions or genetic manipulation (Chambers *et al.,* 2003), embryonic stem cells will rapidly differentiate.

A human embryonic stem cell may be also defined by the presence of several transcription factors and cell surface proteins. The transcription factors Oct4, Nanog, and Sox2 form the core regulatory network that ensures the suppression of genes that lead to differentiation and the maintenance of pluripotency (Boyer *et al.,* 2005). The cell surface antigens most commonly used to identify hES cells include the glycolipids SSEA3 and SSEA4 and the keratan sulfate antigens Tra-1-60 and Tra-1-81.

After twenty years of research, there are no approved treatments or human trials using embryonic stem cells. ES cells, being pluripotent cells, require specific signals for correct differentiation - if injected directly into the body, ES cells will differentiate into many different types of cells, causing a teratoma. Differentiating ES cells into usable cells while avoiding transplant rejection are just a few of the hurdles that embryonic stem cell researchers still face. Many nations currently have moratoria on either ES cell research or the production of new ES cell lines. Because of their combined abilities of unlimited expansion and pluripotency, embryonic stem cells remain a theoretically potential source for regenerative medicine and tissue replacement after injury or disease. However, one way to circumvent these issues is to induce pluripotent status in somatic cells by direct reprogramming.

### C. Induced pluripotent Stem Cells and Reprogramming Factors

Induced pluripotent stem cells, commonly abbreviated as iPS cells or iPSCs, are a type of pluripotent stem cell artificially derived from a non-pluripotent cell, typically an adult somatic cell. Induced pluripotent stem cells are believed to be identical to natural pluripotent stem cells, such as embryonic stem cells in many respects, such as in terms of the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed.

IPS cells were first produced in 2006 (Takahashi *et al.,* 2006) from mouse cells and in 2007 from human cells (Takahashi *et al.,* 2007; *Yu et al,* 2007). This has been cited as an important advancement in stem cell research, as it may allow researchers to obtain pluripotent stem cells, which are important in research and potentially have therapeutic uses, without the controversial use of embryos.

The generation of iPS cells is crucial on the reprogramming factors used for the induction. The following factors or combination thereof could be used in the methods disclosed in the present invention. In certain aspects, nucleic acids encoding Sox and Oct (preferably Oct3/4) will be included into the reprogramming vector. For example, one or more reprogramming vectors may comprise expression cassettes encoding Sox2, Oct4, Nanog and optionally Lin28, or expression cassettes encoding Sox2, Oct4, Klf4 and optionally c-Myc, or expression cassettes encoding Sox2, Oct4, and optionally Esrrb, or expression cassettes encoding Sox2, Oct4, Nanog, Lin28, Klf4, c-Myc, and optionally SV40 Large T antigen. Nucleic acids encoding these reprogramming factors may be comprised in the same expression cassette, different expression cassettes, the same reprogramming vector, or different reprogramming vectors.

Oct4 and certain members of the Sox gene family (Sox1, Sox2, Sox3, and Sox 15) have been identified as crucial transcriptional regulators involved in the induction process whose absence makes induction impossible. Additional genes, however, including certain members of the Klf family (Klf1, Klf2, Klf4, and Klf5), the Myc family (c-Myc, L-Myc, and N-Myc), Nanog, and Lin28, have been identified to increase the induction efficiency.

Oct4 (Pou5f1) is one of the family of octamer ("Oct") transcription factors, and plays a crucial role in maintaining pluripotency. The absence of Oct4 in Oct4⁺ cells, such as blastomeres and embryonic stem cells, leads to spontaneous trophoblast differentiation, and presence of Oct4 thus gives rise to the pluripotency and differentiation potential of embryonic stem cells. Various other genes in the "Oct" family, including Oct4's close relatives, Oct1 and Oct6, fail to elicit induction, thus demonstrating the exclusiveness of Oct-4 to the induction process.

The Sox family of genes is associated with maintaining pluripotency similar to Oct4, although it is associated with multipotent and unipotent stem cells in contrast with Oct4, which is exclusively expressed in pluripotent stem cells. While Sox2 was the initial gene used for induction by *Takahashi et al.* (2006), Wernig *et al.* (2007), and *Yu et al.* (2007), other genes in the Sox family have been found to work as well in the induction process. Sox1 yields iPS cells with a similar efficiency as Sox2, and genes Sox3, Sox15, and Sox18 also generate iPS cells, although with decreased efficiency.

In embryonic stem cells, Nanog, along with Oct4 and Sox2, is necessary in promoting pluripotency. Therefore, it was surprising when Takahashi *et al.* (2006) reported that Nanog was unnecessary for induction although Yu *et al.* (2007) has reported it is possible to generate iPS cells with Nanog as one of the factors.

Lin28 is an mRNA binding protein expressed in embryonic stem cells and embryonic carcinoma cells associated with differentiation and proliferation. Thompson *et al.* demonstrated it is a factor in iPS generation, although it is unnecessary.

Klf4 of the Klf family of genes was initially identified by Takahashi *et al.* (2006) and confirmed by Wernig *et al.* (2007) as a factor for the generation of mouse iPS cells and was demonstrated by *Takahashi et al.* (2007) as a factor for generation of human iPS cells. However, *Yu et al.* (2007) reported that Klf4 was unnecessary for generation of human iPS cells and in fact failed to generate human iPS cells. Klf2 and Klf4 were found to be factors capable of generating iPS cells, and related genes Klf1 and Klf5 did as well, although with reduced efficiency.

The Myc family of genes are proto-oncogenes implicated in cancer. *Takahashi et al.* (2006) and Wernig *et al.* (2007) demonstrated that c-Myc is a factor implicated in the generation of mouse iPS cells and Takahashi *et al.* (2007) demonstrated it was a factor implicated in the generation of human iPS cells. However, Yu *et al.* (2007) and *Takahashi et al.* (2007) reported that c-Myc was unnecessary for generation of human iPS cells. Usage of the "Myc" family of genes in induction of iPS cells is troubling for the eventuality of iPS cells as clinical therapies, as 25% of mice transplanted with c-Myc-induced iPS cells developed lethal teratomas. N-Myc and L-Myc have been identified to induce in the stead of c-myc with similar efficiency. SV40 large antigen may be used to reduce or prevent the cytotoxcity which may occur when c-Myc is expressed.

The reprogramming proteins used in the present invention can be substituted by protein homologs with about the same reprogramming functions. Nucleic acids encoding those homologs could also be used for reprogramming. Conservative amino acid substitutions are preferred--that is, for example, aspartic-glutamic as polar acidic amino acids; lysine/arginine/histidine as polar basic amino acids; leucine/isoleucine/methionine/valine/alanine/glycine/proline as non-polar or hydrophobic amino acids; serine/threonine as polar or uncharged hydrophilic amino acids. Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting polypeptide. Whether an amino acid change results in a functional polypeptide can readily be determined by assaying the specific activity of the polypeptide.

### Reprogramming Factors Expression and Transduction

In certain aspects of the present invention, iPS cells are maded from reprogramming somatic cells using reprogramming factors in combination with genetic modification. The somatic cell in the present invention may be any somatic cell that can be induced to pluripotency, such as a fibroblast, a keratinocyte, a hematopoietic cell, a mesenchymal cell, a liver cell, a stomach cell, or a β cell. In a prefered aspect, T cells may be used as source of somatic cells for reprogramming (see U.S. Application No. 61/184,546).

Reprogramming factors may be expressed from expression cassettes comprised in one or more vectors, such as an integrating vector or an episomal vector. In a further aspect, reprogramming proteins could be introduced directly into somatic cells by protein transduction (see U.S. Application No. 61/172,079).

### A. Integrating Vectors

IPS cells may be derived by transfection of certain nucleic acids or genes encoding reprogramming proteins into non-pluripotent cells, such as T cells, in the present invention. Transfection is typically achieved through integrating viral vectors in the current practice, such as retroviruses. Transfected genes may include the master transcriptional regulators Oct4 (Pouf51) and Sox2, although it is suggested that other genes enhance the efficiency of induction. After a critical period, small numbers of transfected cells may begin to become morphologically and biochemically similar to pluripotent stem cells, and could be isolated through morphological selection, doubling time, or through a reporter gene and antibiotic infection.

In November 2007, a milestone was achieved by creating iPS from adult human fibroblasts from two independent research teams' studies (Yu *et al.,* 2007; Takahashi *et al.,* 2007). With the same principle used earlier in mouse models, Takahashi *et al.* (2007) had successfully transformed human fibroblasts into pluripotent stem cells using the same four pivotal genes: Oct4, Sox2, Klf4, and c-Myc with a retroviral system but c-Myc is oncogenic. *Yu et al.* (2007) used Oct4, Sox2, NANOG, and a different gene LIN28 using a lentiviral system avoiding the use of c-Myc.

As described above, induction of pluripotent stem cells from human dermal fibroblasts has been achieved using retroviruses or lentiviral vectors for ectopic expression of reprogramming genes. Recombinant retroviruses such as the Moloney murine leukemia virus have the ability to integrate into the host genome in a stable fashion. They contain a reverse transcriptase which allows integration into the host genome. Lentiviruses are a subclass of Retroviruses. They are widely adapted as vectors thanks to their ability to integrate into the genome of non-dividing as well as dividing cells. The viral genome in the form of RNA is reverse-transcribed when the virus enters the cell to produce DNA, which is then inserted into the genome at a random position by the viral integrase enzyme. Therefore, successful reprogramming of T cells may use integration-based viral approaches as shown in the Examples section.

### B. Episomal vectors

These reprogramming methods may also make use of extra-chromosomally replicating vectors (*i.e.,* episomal vectors), which are vectors capable of replicating episomally to make iPS cells essentially free of exogenous vector or viral elements (see U.S. Application No. 61/058,858; Yu *et al.,* 2009). A number of DNA viruses, such as adenoviruses, Simian vacuolating virus 40 (SV40) or bovine papilloma virus (BPV), or budding yeast ARS (Autonomously Replicating Sequences)-containing plasmids replicate extra-chromosomally or episomally in mammalian cells. These episomal plasmids are intrinsically free from all these disadvantages (Bode *et al.,* 2001) associated with integrating vectors. For example, a lymphotrophic herpes virus-based episomal vector, including Epstein Barr Virus (EBV) elements as defined below, may replicate extra-chromosomally and help deliver reprogramming genes to somatic cells.

For example, the plasmid-based approach used in the invention may extract robust elements necessary for the successful replication and maintenance of an EBV element-based system without compromising the system's tractability in a clinical setting as described in detail below. The essential EBV elements are OriP and EBNA-1 or their variants or functional equivalents. An additional advantage of this system is that these exogenous elements will be lost with time after being introduced into cells, leading to self-sustained iPS cells essentially free of exogenous elements.

The use of plasmid- or liposome-based extra-chromosomal vectors, e.g., oriP-based vectors, and/or vectors encoding a derivative of EBNA-1 permit large fragments of DNA to be introduced to a cell and maintained extra-chromosomally, replicated once per cell cycle, partitioned to daughter cells efficiently, and elicit substantially no immune response. In particular, EBNA-1, the only viral protein required for the replication of the oriP-based expression vector, does not elicit a cellular immune response because it has developed an efficient mechanism to bypass the processing required for presentation of its antigens on MHC class I molecules (Levitskaya *et al.,* 1997). Further, EBNA-1 can act in *trans* to enhance expression of the cloned gene, inducing expression of a cloned gene up to 100-fold in some cell lines (Langle-Rouault *et al.,* 1998; Evans *et al.,* 1997). Finally, the manufacture of such oriP-based expression vectors is inexpensive.

Other extra-chromosomal vectors include other lymphotrophic herpes virus-based vectors. Lymphotrophic herpes virus is a herpes virus that replicates in a lymphoblast (*e.g.,* a human B lymphoblast) and becomes a plasmid for a part of its natural life-cycle. Herpes simplex virus (HSV) is not a "lymphotrophic" herpes virus. Exemplary lymphotrophic herpes viruses include, but are not limited to EBV, Kaposi's sarcoma herpes virus (KSHV); Herpes virus saimiri (HS) and Marek's disease virus (MDV). Also other sources of episome-base vectors are contemplated, such as yeast ARS, adenovirus, SV40, or BPV.

To circumvent potential problems from viral gene delivery, two groups this year reported on a collaboration that has succeeded in transposon-based approaches for producing pluripotency in human cells without using viral vectors (Woltjen *et al.,* 2009; Kaji *et al.,* 2009). Stable iPS cells were produced in both human and mouse fibroblasts using virus-derived 2A peptide sequences to create a multicistronic vector incorporating the reprogramming factors, delivered to the cell by the piggyBac transposon vector. The 2A-linked reprogramming factors, not required in the established iPS cell lines, were then removed. These strategies could be similarly applied to reprogram T cell in certain aspects of the present invention.

### C. Protein Transduction

One possible way to avoid introducing exogenous genetic modifications to target cells would be to deliver the reprogramming proteins directly into cells, rather than relying on the transcription from delivered genes. Previous studies have demonstrated that various proteins can be delivered into cells *in vitro* and *in vivo* by conjugating them with a short peptide that mediates protein transduction, such as HIV tat and poly-arginine. A recent study demonstrated that murine fibroblasts can be fully reprogrammed into pluripotent stem cells by direct delivery of recombinant reprogramming proteins (Zhou *et al.,* 2009). More details of the methods for reprogramming cells with protein transduction have been disclosed in U.S. Application No. 61/172,079.

In certain aspects of the present invention, protein transduction domains could been used to introduce reprogramming proteins directly into T cells. Protein transduction could be a method for enhancing the delivery of reprogramming proteins into cells. For example, a region of the TAT protein which is derived from the HIV Tat protein can be fused to a target protein allowing the entry of the target protein into the cell. The advantages of using fusions of these transduction domains is that protein entry is rapid, concentration-dependent and appears to work with different cell types.

In a further aspect of the present invention, nuclear localization sequence may also be used to facilitate nuclear entry of reprogramming proteins. Nuclear localization signals (NLS) have been described for various proteins. The mechanism of protein transport to the nucleus is through the binding of a target protein containing a nuclear localization signal to alpha subunit of karyopherin. This is followed by transport of the target protein:karyopherin complex through the nuclear pore and into the nucleus. However, reprogramming proteins are often transcription factors which may have endogenous nuclear localization sequences. Therefore, nuclear localization sequences may not be necessary.

The direct introduction of reprogramming proteins into somatic cells may be used in the present invention, with reprogramming proteins operatively linked to a protein transduction domain (PTD), either by creating a fusion protein comprising such a domain or by chemically cross-linking the reprogramming protein and PTD via functional groups on each molecule.

Standard recombinant nucleic acid methods can be used to express one or more transducible reprogramming proteins used herein. In one embodiment, a nucleic acid sequence encoding the transducible protein is cloned into a nucleic acid expression vector, e.g., with appropriate signal and processing sequences and regulatory sequences for transcription and translation. In another embodiment, the protein can be synthesized using automated organic synthetic methods.

In addition, there have been several methods that may also help the transport of proteins into cells, one ore more of which can be used alone or in combination with the methods using the protein transduction domains, including, but not limited to, microinjection, electroporation, and the use of liposomes. Most of these methods may need a purified preparation of protein. Purification of recombinant proteins is often facilitated by the incorporation of an affinity tag into the expression construct, making the purification step fast and efficient.

### IPS Cells Selection, Culturing, and Differentiation

In certain aspects of the invention, after one or more reprogramming factors are introduced into somatic cells, cells will be cultured for expansion (optionally selected for the presence of vector elements like positive selection or screenable marker to concentrate transfected cells). Reprogramming vectors may express reprogramming factors in these cells and replicate and partition along with cell division. Alternatively, reprogramming proteins could enter these cells and their progeny by replenishing medium containing the reprogramming proteins. These reprogramming factors will reprogram somatic cell genome to establish a self-sustaining pluripotent state, and in the meantime or after removal of positive selection of the presence of vectors, exogenous genetic elements will be lost gradually, or there is no need to add reprogramming proteins.

These induced pluripotent stem cells could be selected from progeny cells based on embryonic stem cell characteristics because they are expected to be substantially identical to pluripotent embryonic stem cells. An additional negative selection step could be also employed to accelerate or help selection of iPS cells essentially free of exogenous genetic elements by testing the absence of reprogramming vector DNA or using selection markers, such as reporters.

### A. IPS Cell Selection

The successfully generated iPSCs from previous studies were remarkably similar to naturally-isolated pluripotent stem cells (such as mouse and human embryonic stem cells, mESCs and hESCs, respectively) in the following respects, thus confirming the identity, authenticity, and pluripotency of iPSCs to naturally-isolated pluripotent stem cells. Thus, induced pluripotent stem cells generated from the methods disclosed herein could be selected based on one or more of following embryonic stem cell characteristics.

### i. Cellular biological properties

**Morphology:** iPSCs are morphologically similar to ESCs. Each cell may have round shape, dual nucleoli or large nucleolus and scant cytoplasm. Colonies of iPSCs could also be similar to that of ESCs. Human iPSCs form sharp-edged, flat, tightly-packed colonies similar to hESCs and mouse iPSCs form colonies similar to mESCs, which are less flat and more aggregated colonies than that of hESCs.

**Growth properties:** Doubling time and mitotic activity are cornerstones of ESCs, as stem cells must self-renew as part of their definition. iPSCs could be mitotically active, actively self-renewing, proliferating, and dividing at a rate equal to ESCs.

**Stem Cell Markers:** iPSCs may express cell surface antigenic markers expressed on ESCs. Human iPSCs expressed the markers specific to hESC, including, but not limited to, SSEA-3, SSEA-4, TRA-1-60, TRA-1-81, TRA-2-49/6E, and Nanog. Mouse iPSCs expressed SSEA-1 but not SSEA-3 nor SSEA-4, similarly to mESCs.

**Stem Cell Genes:** iPSCs may express genes expressed in undifferentiated ESCs, including Oct4, Sox2, Nanog, GDF3, REX1, FGF4, ESG1, DPPA2, DPPA4, and hTERT.

**Telomerase Activity:** Telomerases are necessary to sustain cell division unrestricted by the Hayflick limit of ∼50 cell divisions. hESCs express high telomerase activity to sustain self-renewal and proliferation, and iPSCs also demonstrate high telomerase activity and express hTERT (human telomerase reverse transcriptase), a necessary component in the telomerase protein complex.

**Pluripotency:** iPSCs will be capable of differentiation in a fashion similar to ESCs into fully differentiated tissues.

**Neural Differentiation:** iPSCs could be differentiated into neurons, expressing βIII-tubulin, tyrosine hydroxylase, AADC, DAT, ChAT, LMX1B, and MAP2. The presence of catecholamine-associated enzymes may indicate that iPSCs, like hESCs, may be differentiable into dopaminergic neurons. Stem cell-associated genes will be downregulated after differentiation.

**Cardiac Differentiation:** iPSCs could be differentiated into cardiomyocytes that spontaneously begin beating. Cardiomyocytes express cTnT, MEF2C, MYL2A, MHCβ, and NKX2.5. Stem cell-associated genes will be downregulated after differentiation.

**Teratoma Formation:** iPSCs injected into immunodeficient mice may spontaneously form teratomas after certain time, such as nine weeks. Teratomas are tumors of multiple lineages containing tissue derived from the three germ layers endoderm, mesoderm and ectoderm; this is unlike other tumors, which typically are of only one cell type. Teratoma formation is a landmark test for pluripotency.

**Embryoid Body:** hESCs in culture spontaneously form ball-like embryo-like structures termed "embryoid bodies," which consist of a core of mitotically active and differentiating hESCs and a periphery of fully differentiated cells from all three germ layers. iPSCs may also form embryoid bodies and have peripheral differentiated cells.

**Blastocyst Injection:** hESCs naturally reside within the inner cell mass (embryoblast) of blastocysts, and in the embryoblast, differentiate into the embryo while the blastocyst's shell (trophoblast) differentiates into extraembryonic tissues. The hollow trophoblast is unable to form a living embryo, and thus it is necessary for the embryonic stem cells within the embryoblast to differentiate and form the embryo. iPSCs injected by micropipette into a trophoblast to generate a blastocyst transferred to recipient females, may result in chimeric living mouse pups: mice with iPSC derivatives incorporated all across their bodies with 10%-90 and chimerism.

### ii. Epigenetic reprogramming

**Promoter Demethylation:** Methylation is the transfer of a methyl group to a DNA base, typically the transfer of a methyl group to a cytosine molecule in a CpG site (adjacent cytosine/guanine sequence). Widespread methylation of a gene interferes with expression by preventing the activity of expression proteins or recruiting enzymes that interfere with expression. Thus, methylation of a gene effectively silences it by preventing transcription. Promoters of pluripotency-associated genes, including Oct4, Rex1, and Nanog, may be demethylated in iPSCs, showing their promoter activity and the active promotion and expression of pluripotency-associated genes in iPSCs.

**Histone Demethylation:** Histones are compacting proteins that are structurally localized to DNA sequences that can effect their activity through various chromatin-related modifications. H3 histones associated with Oct/4, Sox2, and Nanog may be demethylated to activate the expression of Oct4, Sox2, and Nanog.

### B. Culturing and Differentiation of iPS cells

After somatic cells are introduced with reprogramming factors using the disclosed methods, these cells may be cultured in a medium sufficient to maintain the pluripotency. Culturing of induced pluripotent stem (iPS) cells generated in this invention can use various medium and techniques developed to culture primate pluripotent stem cells, more specially, embryonic stem cells, as described in U.S. Pat. App. 20070238170 and U.S. Pat. App. 20030211603. It is appreciated that additional methods for the culture and maintenance of human pluripotent stem cells, as would be known to one of skill, may be used with the present disclosure.

In certain embodiments, undefined conditions may be used; for example, pluripotent cells may be cultured on fibroblast feeder cells or a medium which has been exposed to fibroblast feeder cells in order to maintain the stem cells in an undifferentiated state. Alternately, pluripotent cells may be cultured and maintained in an essentially undifferentiated state using defined, feeder-independent culture system, such as a TeSR medium (Ludwig *et al*., 2006a; Ludwig *et al*., 2006b). Feeder-independent culture systems and media may be used to culture and maintain pluripotent cells. These approaches allow human embryonic stem cells to remain in an essentially undifferentiated state without the need for mouse fibroblast "feeder layers." As described herein, various modifications may be made to these methods in order to reduce costs as desired.

For example, like human embryonic stem (hES) cells, iPS cells can be maintained in 80% DMEM (Gibco #10829-018 or #11965-092), 20% defined fetal bovine serum (FBS) not heat inactivated (or human AB serum), 1% non-essential amino acids, 1 mM L-glutamine, and 0.1 mM β-mercaptoethanol. Alternatively, iPS cells can be maintained in serum-free medium, made with 80% Knock-Out DMEM (Gibco #10829-018), 20% serum replacement (Gibco #10828-028), 1% non-essential amino acids, 1 mM L-glutamine, and 0.1 mM β-mercaptoethanol. Just before use, human bFGF may be added to a final concentration of about 4 ng/mL (WO 99/20741) or zebrafish bFGF may be used instead as in the Examples.

Various matrix components may be used in culturing and maintaining human pluripotent stem cells. For example, collagen IV, fibronectin, laminin, and vitronectin in combination may be used to coat a culturing surface as a means of providing a solid support for pluripotent cell growth, as described in Ludwig *et al.* (2006a; 2006b), which are incorporated by reference in its entirety.

Matrigel^{™} may also be used to provide a substrate for cell culture and maintenance of human pluripotent stem cells. Matrigel^{™} is a gelatinous protein mixture secreted by mouse tumor cells and is commercially available from BD Biosciences (New Jersey, USA). This mixture resembles the complex extracellular environment found in many tissues and is used by cell biologists as a substrate for cell culture.

IPS cells, like ES cells, have characteristic antigens that can be identified or confirmed by immunohistochemistry or flow cytometry, using antibodies for SSEA-1, SSEA-3 and SSEA-4 (Developmental Studies Hybridoma Bank, National Institute of Child Health and Human Development, Bethesda Md.), and TRA-1-60 and TRA-1-81 (Andrews *et al*., 1987). Pluripotency of embryonic stem cells can be confirmed by injecting approximately 0.5-10 X 10⁶ cells into the rear leg muscles of 8-12 week old male SCID mice. Teratomas develop that demonstrate at least one cell type of each of the three germ layers.

Various approaches may be used with the present disclosure to differentiate genetically modified iPS cells into cell lineages including, but not limited to, hematopoietic cells, myocytes (*e.g.*, cardiomyocytes), neurons, fibroblasts and epidermal cells, and tissues or organs derived therefrom. Hematopoietic differentiation may be preferred. Exemplary methods of hematopoietic differentiation of iPS cells may include, but are not limited to, methods disclosed by U.S. Application No. 61/088,054 and No. 61/156,304, or embryoid body (EB) based methods (Chadwick *et al*., 2003; Ng *et al*., 2005). Fibronectin differentiation methods may also be used for blood lineage differentiation, as exemplified in Wang *et al*., 2007.

### Modification of Gene Structure

The generation of null alleles or other genetic alterations has been a powerful technique that allows the assessment of gene function and molecular mechanisms. Certain embodiments of the present invention provide genetically modified stem cells, such as induced pluripotent stem cells, that have a mutant gene structure related to HIV infection, particularly HIV cellular entry. Genetic modification can be applied before, during, or after reprogramming.

For example, gene targeting approach can be the inactivation of all alleles to generate a knockout cell line or introduce sequences into an endogenous gene, thereby creating one or more knockin alleles. Several methods have been developed for genetic modification, including homologous recombination and nuclease editing, which may be suitable for stem cells.

### A. Homologous Recombination

To achieve targeted, as opposed to random, delivery of a genetic construct into the genome of stem cells, homologous recombination may be used to target the delivery. To accomplish the objective of making and identifying homologous recombination events in human stem cells, a transfection technique could be used that is efficient enough to permit the identification and recovery of cells in which the homologous recombination events has occurred. Since homologous recombination events can sometimes occur at low frequencies, relatively high efficiency in the transfection method is needed so that large numbers of cells could be conveniently transfected at reasonable efficiencies.

In certain aspects of the present invention, techniques that have been used to transform human ES cells could be used in combination with reprogramming to make modified iPS cells. Various research groups have reported attempts to transform human ES cells with liposome-based techniques. Recently there has been reported a successful gene targeting methodology which makes use of homologous recombination, in conjunction with a modified electroporation technique, and that combination has proved effective at reasonable efficiency to achieve directed genetic transformations of human embryonic cell lines (Zwaka and Thomson, 2003). Two important attributes of the method described in the report are the use of electroporation to introduce the genetic construct into the ES cell and homologous recombination to facilitate introduction of the genetic construct into a desired target location in the genome of the ES cells. The use of this modified electroporation technique permits ES cells to be transfected by foreign DNA at reasonable efficiencies. This technique has been modified from the technique used with murine embryonic stem cells, and achieves better results in human and primate ES cells than can be achieved with the murine technique. It has been demonstrated that electroporation with homologous recombination can be used in human ES cells to achieve directed or targeted gene insertion in living human ES cells. Homologous recombination events offer a distinct advantage over random gene insertions in that the site of the insertion of foreign DNA can be controlled, thus avoiding unwanted gene insertion and permitting targeted manipulation of native genes.

The genetic construct useful for homologous recombination may include homologous arms and a delivered genetic insert. There may be two such homologous arms, 3' and 5' homologous arms. The 3' and 5' homologous arm segments or regions are constructed to be identical in sequence to native genomic DNA sequences in regions of the genome 3' and 5' of the location where the genetic insert is to be inserted. In this way, by native cellular processes, the 3' and 5' homologous arms recombine with the corresponding native segment of DNA in the target site in the genome, thereby transferring into the genome the delivered genetic insert and removing the native DNA between the 3' and 5' native genomic segments. This process may happen naturally using native cellular factors, but at low frequency.

Gene targeting by homologous recombination may be generally useful for making many kinds of targeted genetic transformations in successor cell cultures or populations made from primate and human iPS cells in culture. Gene targeting can be used to make either "knock-out" or "knock-in" stem cell cultures, including iPS cells. If it is desired to produce a cell line in which a selected native gene in the iPS cells is silenced or disrupted, this can be done by making a "knock-out" genetic construct. In this alternative, the delivered genetic insert can be, in essence, no DNA at all, but the knock-out insertion is preferably a DNA sequence which simply does not encode a gene product at all. In knock-out cells, the functioning of a particular targeted native gene is disrupted or suppressed in the genome of those cells, such as a CCR5 null mutant. This could be done by replacing the native genetic sequence by homologous recombination with a genetic sequence that does not express the same protein or nucleotide as the sequence replaced.

If the genetic insert is intended to produce a gene product, the genetic insert should be a construction capable of expressing a gene product in an iPS cell. This alternative is sometimes referred to here as the "knock-in" approach, by which a previously constructed genetic insert, producing a gene product, is substituted for a genetic sequence previously in the cells. The gene product would typically be a protein, but the production of other gene products such as RNAs (including interfering RNAs and antisense RNAs) is also contemplated. To produce a gene product, the genetic insert would typically be an expression cassette including, in sequence, a promoter, a coding sequence for the gene product and a transcriptional terminator sequence, all selected to be effective in the iPS cells and appropriate for the overall process being performed.

The knock-in alternative also offers a powerful way to offer the ability to create cultures of differentiated cells directly from iPS cells. To do this, preferably the expression cassette in the genetic insert includes a promoter which drives the expression of a screenable marker gene or selectable marker gene coding sequence which is positioned behind the promoter in the genetic construct. The promoter is a tissue specific promoter that only drives expression of the screenable or selectable marker if the iPS cell into which the expression cassette has been transformed has then later differentiated into a selected cell lineage. For example, if the promoter is specific to a type of hematopoietic cells, the promoter would become active to drive its associated gene expression only in those iPS-derived cells which have differentiated into the type of hematopoietic cells. If the gene driven by the tissue specific promoter is a selectable marker, it can be used to select for cells which have undergone the desired differentiation.

An alternative strategy is to make a gene expression construct without promoters of any kind, and then to insert the construct into the genome of iPS cells in a site where the genetic construct will only be expressed by native promoter activity in the cells which is specific to a desired state lineage or state of differentiation. This promoter activity would be chosen to be a promoter which is active only when the cells are in a desired differentiation lineage.

Again, a screenable marker or selectable marker gene coding sequence is useful to distinguish the cells which have achieved the selected state of differentiation from other cells in culture. A screenable marker gene would be a gene the expression of which can be observed in a living cell, such as the green fluorescent protein (GFP) or luciferase, but which cannot be used to kill non-transformed cells. A screenable marker gene is used to identify transformed cells expressing the marker through visible cell selection techniques, such as fluorescent cell sorting techniques. A selectable marker would be a gene that confers resistance to a selection agent, such as antibiotic resistance, which is lethal to cells not having the selectable marker. A selectable marker is used in conjunction with a selection agent to select in culture for cells expressing the inserted gene construct.

### B. Zinc Finger Nucleases

The inventors also contemplate using zinc finger nucleases for making genetically modified iPS cells. Zinc finger nucleases (ZFNs) are artificial restriction enzymes generated by fusing a zinc finger DNA-binding domain to a DNA-cleavage domain. Zinc finger domains can be engineered to target desired DNA sequences which enables zinc finger nucleases to target unique sequence within a complex genome. By taking advantage of endogenous DNA repair machinery, these reagents can be used to precisely alter the genomes of higher organisms. Recently zinc finger nucleases were reported for genome modification in human embryonic stem cells (Lombardo et al., 2007; U.S. Publ. 2009/0117617).

For example, an integrase-defective lentiviral vector (IDLV) comprising a desired gene structure relevant to HIV cellular entry may be integrated in a site-specific manner (targeted integration) using zinc finger nucleases (ZFNs). ZFNs comprise a zinc finger protein (ZFP) and a nuclease (cleavage) domain. Zinc finger binding domains can be engineered to bind to a sequence of choice. See, for example, Beerli *et al.* (2002); Pabo *et al.* (2001); Isalan *et al.* (2001); Segal *et al.* (2001); Choo *et al.* (2000).

An engineered zinc finger binding domain can have a novel binding specificity, compared to a naturally-occurring zinc finger protein. Engineering methods include, but are not limited to, rational design and various types of selection. Rational design includes, for example, using databases comprising triplet (or quadruplet) nucleotide sequences and individual zinc finger amino acid sequences, in which each triplet or quadruplet nucleotide sequence is associated with one or more amino acid sequences of zinc fingers which bind the particular triplet or quadruplet sequence. See, for example, co-owned U.S. Pat. Nos. 6,453,242 and 6,534,261.

Exemplary selection methods, including phage display and two-hybrid systems, are disclosed in U.S. Pat. Nos. 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237. Enhancement of binding specificity for zinc finger binding domains has been described, for example, in WO 02/077227.

ZFPs and methods for design and construction of fusion proteins (and polynucleotides encoding same) are known to those of skill in the art and described in detail in related to U.S. Publication Nos. 20030232410; 20050208489; 2005064474; 20050026157; 20060188987; International Publication WO 07/014,275; U.S. patent application Ser. Nos. 10/587,723 (filed Jul. 27, 2006); 11/493,423 (filed Jul. 26, 2006).

In certain embodiments, the ZFNs described herein are carried on an adenovirus vector, for example the chimeric Ad5/35 vector. As noted herein, the ZFNs may comprise 2, 3, 4, 5, 6 or even more zinc finger domains.

A ZFP binding domain may be fused to a cleavage domain or cleavage half-domain of a nuclease. In certain embodiments, the ZFP is fused to a cleavage half-domain of a Type IIs restriction endonuclease, for example FokI. When fused to a cleavage half-domain, a pair of such zinc finger/nuclease half-domain fusions are used for targeted cleavage, as disclosed, for example, in U.S. Patent Publication No. 20050064474. For targeted cleavage, the near edges of the binding sites can separated by 5 or more nucleotide pairs, and each of the fusion proteins can bind to an opposite strand of the DNA target.

As noted above, the ZFNs may also comprise a nuclease (cleavage domain, cleavage half-domain). The cleavage domain portion of the fusion proteins disclosed herein can be obtained from any endonuclease or exonuclease. Exemplary endonucleases from which a cleavage domain can be derived include, but are not limited to, restriction endonucleases and homing endonucleases. See, for example, 2002-2003 Catalogue, New England Biolabs, Beverly, Mass.; and Belfort *et al.* (1997). Additional enzymes which cleave DNA are known (*e.g.,* S1 Nuclease; mung bean nuclease; pancreatic DNase I; micrococcal nuclease; yeast HO endonuclease; see also Linn *et al*., 1993). One or more of these enzymes (or functional fragments thereof) can be used as a source of cleavage domains and cleavage half-domains.

Similarly, a cleavage half-domain can be derived from any nuclease or portion thereof, as set forth above, that requires dimerization for cleavage activity. In general, two fusion proteins are required for cleavage if the fusion proteins comprise cleavage half-domains. Alternatively, a single protein comprising two cleavage half-domains can be used. The two cleavage half-domains can be derived from the same endonuclease (or functional fragments thereof), or each cleavage half-domain can be derived from a different endonuclease (or functional fragments thereof). In addition, the target sites for the two fusion proteins are preferably disposed, with respect to each other, such that binding of the two fusion proteins to their respective target sites places the cleavage half-domains in a spatial orientation to each other that allows the cleavage half-domains to form a functional cleavage domain, *e.g.,* by dimerizing. Thus, in certain embodiments, the near edges of the target sites are separated by 5-8 nucleotides or by 15-18 nucleotides. However any integral number of nucleotides or nucleotide pairs can intervene between two target sites *(e.g.,* from 2 to 50 nucleotide pairs or more). In general, the site of cleavage lies between the target sites.

Restriction endonucleases (restriction enzymes) are present in many species and are capable of sequence-specific binding to DNA (at a recognition site), and cleaving DNA at or near the site of binding. Certain restriction enzymes (*e.g.,* Type IIS) cleave DNA at sites removed from the recognition site and have separable binding and cleavage domains. For example, the Type IIS enzyme Fok I catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li *et al.* (1992); Li *et al.* (1993); Kim *et al.* (1994a); Kim *et al.* (1994b). Thus, in one embodiment, fusion proteins comprise the cleavage domain (or cleavage half-domain) from at least one Type IIS restriction enzyme and one or more zinc finger binding domains, which may or may not be engineered.

An exemplary Type IIS restriction enzyme, whose cleavage domain is separable from the binding domain, is Fok I. This particular enzyme is active as a dimer. Bitinaite *et al.* (1998). Accordingly, for the purposes of the present disclosure, the portion of the Fok I enzyme used in the disclosed fusion proteins is considered a cleavage half-domain. Thus, for targeted double-stranded cleavage and/or targeted replacement of cellular sequences using zinc finger-Fok I fusions, two fusion proteins, each comprising a FokI cleavage half-domain, can be used to reconstitute a catalytically active cleavage domain. Alternatively, a single polypeptide molecule containing a zinc finger binding domain and two Fok I cleavage half-domains can also be used. Parameters for targeted cleavage and targeted sequence alteration using zinc finger-Fok I fusions are provided elsewhere in this disclosure.

A cleavage domain or cleavage half-domain can be any portion of a protein that retains cleavage activity, or that retains the ability to multimerize (*e.g*., dimerize) to form a functional cleavage domain.

Exemplary Type IIS restriction enzymes are described in International Publication WO 07/014,275. Additional restriction enzymes also contain separable binding and cleavage domains, and these are contemplated by the present disclosure. See, for example, Roberts *et al.* (2003).

In certain embodiments, the cleavage domain comprises one or more engineered cleavage half-domain (also referred to as dimerization domain mutants) that minimize or prevent homodimerization, as described, for example, in U.S. Patent Publication Nos. 20050064474 and 20060188987 (application Ser. Nos. 10/912,932 and 11/304,981, respectively) and in U.S. provisional patent application No. 60/808,486 (filed May 25, 2006). Amino acid residues at positions 446, 447, 479, 483, 484, 486, 487, 490, 491, 496, 498, 499, 500, 531, 534, 537, and 538 of FokI are all targets for influencing dimerization of the Fok I cleavage half-domains.

Engineered cleavage half-domains described herein can be prepared using any suitable method, for example, by site-directed mutagenesis of wild-type cleavage half-domains (Fok I) as described in U.S. Patent Publication No. 20050064474 (Ser. No. 10/912,932).

Any nuclease having a target site in the target gene can be used in the methods disclosed herein. For example, homing endonucleases and meganucleases have very long recognition sequences, some of which are likely to be present, on a statistical basis, once in a human-sized genome. Any such nuclease having a unique target site in a target gene can be used instead of, or in addition to, a zinc finger nuclease, for targeted cleavage in a target gene.

Exemplary homing endonucleases include I-SceI, I-CeuI, PI-PspI, PI-Sce, I-SceIV, I-CsmI, I-PanI, I-SceII, I-PpoI, I-SceIII, I-CreI, I-TevI, I-TevII and I-TevIII. Their recognition sequences are known. See also U.S. Pat. No. 5,420,032; U.S. Pat. No. 6,833,252; Belfort *et al.* (1997); Dijon *et al.* (1989); Perler *et al.* (1994); Jasin (1996); Gimble *et al.* (1996); Argast *et al.* (1998) and the New England Biolabs catalogue.

Although the cleavage specificity of most homing endonucleases is not absolute with respect to their recognition sites, the sites are of sufficient length that a single cleavage event per mammalian-sized genome can be obtained by expressing a homing endonuclease in a cell containing a single copy of its recognition site. It has also been reported that the specificity of homing endonucleases and meganucleases can be engineered to bind non-natural target sites. See, for example, Chevalier *et al.* (2002); Epinat *et al.* (2003); Ashworth *et al.* (2006); Paques *et al.* (2007).

### Therapeutic Applications

Highly active antiretroviral therapy prolongs the life of HIV-infected individuals, but it requires lifelong treatment and results in cumulative toxicities and viral-escape mutants. Genetically modified iPS cells of the present invention offer the promise of preventing progressive HIV infection by sustained interference with viral replication, alone or in combination with less toxic chronic chemotherapy. In certain embodiments, the cells containing the desired modified gene structure may be transplanted without HLA typing, preferably, in the case of the donor of the somatic cells for making the iPS cells and the recipient are identical or HLA-type matched, or alternatively, the recipients are immunocompromised. In other embodiments, the cells are HLA typed to ensure compatibility with the recipient.

In certain embodiments, the genetically modified iPS cells may be transplanted. The normal stem cell population (which ultimately produces the lymphocytes susceptible to viral replication) could be eliminated or reduced prior to transplantation of the therapeutic stem cell units. Chemotherapy, radiation, or the techniques described in U.S. Pat. No. 6,217,867 are used to condition the bone marrow for appropriate engraftment of the transplant. Finally, therapeutic stem cell units comprising the desired modified gene structure are transplanted into the patient using standard methods.

In some embodiments, the isolated population of cells comprising desired gene structure also comprise a pharmaceutical carrier, preferably an aqueous carrier. A variety of aqueous carriers can be used, *e.g*., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate, albumin, anticoagulants such as CPD (citrate, phosphate, and dextrose), dextran, DMSO, combinations thereof, and the like. The concentration of active agent in these formulations can vary widely, and will be selected primarily based on fluid volumes, viscosities, body weight and the like in accordance with the particular mode of administration selected and the patient's needs.

Preferably, the methods and cells of this disclosure can be used to treat or prevent any disease or condition that arises from HIV infection, such as AIDS and ARC. It should be recognized that methods of this invention can easily be practiced in conjunction with existing antiviral therapies to effectively treat or prevent disease.

The progression of HIV disease may influence the selection of cell types derived from iPS cells for transplant. Briefly, early in infection HIV isolates are predominantly moncytotropic (M-tropic), that is their host range is generally limited to PBLs and cells of the monocyte/macrophage lineage. M-tropic HIV strains infect macrophages via CCR5. As infection progresses HIV isolates become increasingly T-cell-line tropic (TCL tropic), that is their host range is generally limited to T-cells. TCL-tropic HIV strains infect T-cells via CXCR4. Dual tropic HIV isolates are also known. Those of skill will recognize that the tropism of a particular HIV isolate can be determined by assaying the ability of an HIV strain to infect a cell line. That is, an M-tropic HIV strain will be able to infect a macrophage line, while a TCL tropic HIV strain will be able to infect a T-cell line. In addition, TCL tropic HIV strains cause syncytia formation after infection and this can be detected by those of skill.

In some embodiments, iPS cells or cells of the monocyte/macrophage lineage derived therefrom with beneficial CCR5 polymorphisms may be transplanted into patients infected with predominantly M-tropic HIV viruses. Beneficial CCR5 polymorphisms can also be used to treat patients with a mixture of M-tropic and TCL-tropic HIV strains, or dual tropic HIV strains. In some other embodiments, iPS cells or T cells with beneficial CXCR4 polymorphisms derived therefrom are transplanted into patients infected with predominantly TCL-tropic HIV viruses. Beneficial CXCR4 polymorphisms can also be used to treat patients with a mixture of M-tropic and TCL-tropic HIV strains, or dual tropic HIV strains.

Factors and events which form a theoretical basis for the embodiments of the invention are discussed herein.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1 - Gene Targeting of iPS Cells

A gene-targeting vector is constructed by replacement of the DNA sequence encoding the second transmembrane domain of the CCR5 co-receptor with a mutant form of the second transmembrane domain harboring a 32-bp deletion mimicking the CCR5 delta32 mutant and a *neo* cassette. The mutant form is flanked by two homologous arms that are identical to surrounding segments of the CCR5 gene. Isogenic homologous DNA is obtained by long-distance genomic PCR and subcloned.

Human iPS cell lines are obtained by the lentiviral-mediated transduction of four transcription factors (OCT4, SOX2, NANOG and LIN28) as previously described (Yu *et al*., 2007). The iPS cells are maintained on Matrigel^{™} and cultured in TeSR medium.

A single cell suspension is made by incubating colonies in TrypLE, a recombinant trypsin-like enzyme (Invitrogen, Carlsbad, CA) at 37 degrees for 7 minutes, washed twice with TeSR medium containing the apoptosis inhibitor H1152 (or the myosin II ATPase inhibitor blebbistatin) and soybean trypsin inhibitor, and resuspended in 0.5 ml of cold PBS containing H1152 (or blebbistatin) and soybean trypsin inhibitor. For electroporation, 0.3 ml PBS (Invitrogen) containing 40 µg linearized targeting vector are added to the iPS cells and incubated on ice for 5 minutes. The iPS cells are then exposed to a single 320V, 200 µF pulse at room temperature using the BioRad Gene Pulser Xcell (0.4 cm gap cuvette; BioRad, Hercules, CA). Cells are washed in 2 ml of TeSR medium containing H1152 (or blebbistatin) and soybean trypsin inhibitor and plated in 5 six well plate dishes coated with Matrigel. Electroporated cells are maintained in TeSR medium containing only H1152 (or blebbistatin).

G418 selection (50 µg/ml) is started 48 hours after electroporation and H1152 (or blebbistatin) is removed from the medium. After two weeks, surviving colonies are analyzed individually by PCR using primer specific for the *neo* cassette and for the CCR5 32 bp deletion, respectively. PCR-positive clones are re-screened by Southern blot analysis using *Pst*I-digested DNA and a probe on the 3' side of the *neo* cassette.

All of the methods disclosed and claimed herein can be made and executed without undue experimentation in light of the present disclosure. While the compositions and methods of this disclosure have been described in terms of preferred embodiments, it will be apparent to those of skill in the art that variations may be applied to the methods and in the steps or in the sequence of steps of the method described herein without departing from the concep and scope of the disclosure. More specifically, it will be apparent that certain agents which are both chemically and physiologically related may be substituted for the agents described herein while the same or similar results would be achieved. All such similar substitutes and modifications apparent to those skilled in the art are deemed to be within the scope and concept of the disclosure.

### REFERENCES

U.S. Patent 5,356,802
U.S. Patent 5,420,032
U.S. Patent 5,436,150
U.S. Patent 5,487,994
U.S. Patent 5,789,538
U.S. Patent 5,925,523
U.S. Patent 6,007,988
U.S. Patent 6,013,453
U.S. Patent 6,140,466
U.S. Patent 6,153,431
U.S. Patent 6,200,759
U.S. Patent 6,217,867
U.S. Patent 6,242,568
U.S. Patent 6,410,248
U.S. Patent 6,453,242
U.S. Patent 6,534,261
U.S. Patent 6,833,252
U.S. Patent Appln. 10/587,723
U.S. Patent Appln. 11/493,423
U.S. Patent Publn. 20030211603
U.S. Patent Publn. 20030232410
U.S. Patent Publn. 20050026157
U.S. Patent Publn. 20050064474
U.S. Patent Publn. 20050064474
U.S. Patent Publn. 20050208489
U.S. Patent Publn. 20050064474
U.S. Patent Publn. 20060188987
U.S. Patent Publn. 20070238170
U.S. Patent Publn. 2009/0117617
U.S. Patent Appln. Serial 60/808,486
U.S. Patent Appln. Serial 61/058,858
U.S. Patent Appln. Serial 61/088,054
U.S. Patent Appln. Serial 61/156,304
U.S. Patent Appln. Serial 61/172,079
U.S. Patent Appln. Serial 61/184,546
Andrews et al., In: Teratocarcinomas and Embryonic Stem Cells, Robertson (Ed.), IRL Press, 207-246,1987.
Argast et al., J. Mol. Biol., 280:345-353, 1998.
Ashworth et al., Nature, 441:656-659, 2006.
Beerli et al., Nature Biotechnol., 20:135-141, 2002.
Belfort et al., Nucleic Acids Res., 25:3379-3388, 1997.
Belfort et al., Nucleic Acids Res., 25:3379-3388, 1997.
Bitinaite et al., Proc. Natl. Acad. Sci. USA, 95:10,570-10,575, 1998.
Bode et al., Gene Ther. Mol. Biol., 6:33-46, 2001.
Boyer et al., Cell, 122(6):947-56, 2005.
Catalogue, New England Biolabs, Beverly, Mass, 2002-2003.
Chadwick et al., Blood, 102(3):906-15, 2003.
Chambers et al., Cell, 113(5):643-55, 2003.
Chevalier et al., Molec. Cell, 10:895-905, 2002.
Choo et al., Curr. Opin. Struct. Biol., 10:411-416, 2000.
Clapham and Weiss, Nature, 388:230-231, 1997.
Clegg et al. AIDS, 14:103-108, 2000.
Coffin et al., Nature, 321(6065):10, 1986.
Dean et al. Science, 273:1856-1862, 1996.
Dijon et al., Gene, 82:115-118, 1989.
Epinat et al., Nucleic Acids Res., 31:2952-2962, 2003.
Evans, et al., In: Cancer Principles and Practice of Oncology, Devita et al. (Eds.), Lippincot-Raven, NY, 1054-1087, 1997.
Flores-Villanueva et al., Proc. Natl. Acad. Sci. USA, 98:5140-5145, 2001.
GB 2,338,237
Gimble et al., J. Mol. Biol., 263:163-180, 1996.
Hogan et al. Ann. Intern. Med., 134:978-996, 2001.
Hütter et al., N. Engl. J. Med., 360(7):692-8, 2009.
Isalan et al., Nature Biotechnol., 19:656-660, 2001.
Jasin, Trends Genet., 12:224-228, 1996.
Kaji et al., Nature, 458, 771-775, 2009.
Kim et al., J. Biol. Chem., 269:31,978-31,982, 1994b.
Kim et al., Proc. Natl. Acad. Sci. USA, 91:883-887, 1994a.
Kroner et al. AIDS, 9:275-280, 1995.
Langle-Rouault et al., J. Virol., 72(7):6181-6185, 1998.
Levitskaya et al., Proc. Natl. Acad. Sci. USA, 94(23):12616-12621, 1997.
Li et al., Proc. Natl. Acad. Sci. USA, 89:4275-4279, 1992.
Li et al., Proc. Natl. Acad. Sci. USA, 90:2764-2768, 1993.
Linn et al., In: Nucleases, Cold Spring Harbor Laboratory Press, 1993.
Liu et al. Cell, 86:367-377, 1996.
Liu et al., Proc. Natl. Acad. Sci. USA, 96:4581-4585, 1999.
Lombardo et al., Nature Biotechnology, 25:1298-1306, 2007.
Ludwig et al., Nat. Biotechnol., 24(2):185-187, 2006b.
Ludwig et al., Nat. Methods, 3(8):637-46, 2006a.
Makino et al. J. Immunol., 144:4347-4355, 1990.
Martin et al., Infectious Disease, 282:1907-1911, 1998.
New England Biolabs Catalogue
Ng et al., Development, 132(5):873-84, 2005.
O'Brien et al., Hospital Practise, July 15, 1998.
Ometto et al. J. Infectious Disease, 183:814-818, 2001.
Pabo et al., Ann. Rev. Biochem., 70:313-340, 2001.
Paques et al., Currant Gene Therapy, 7:49-66, 2007.
PCT Appln. WO 00/27878
PCT Appln. WO 01/88197
PCT Appln. WO 02/077227
PCT Appln. WO 07/014,275
PCT Appln. WO 07/014,275
PCT Appln. WO 98/37186
PCT Appln. WO 98/53057
PCT Appln. WO 99/20741
Perler et al., Nucleic Acid Res., 22:1125-1127, 1994.
Quillent et al. Lancet, 351:14-18, 1998.
Roberts et al., Nucleic Acids Res., 31:418-420, 2003.
Roger et al. FASEB, 12:625-632, 1998.
Samson et al., Nature, 382(6593):722-5, 1996.
Segal et al., Curr. Opin. Biotechnol., 12:632-637, 2001.
Shin et al., Proc. Natl. Acad. Sci. USA, 97:14467-72, 2000.
Smith et al. Science, 277:959-965, 1997.
Sowadsky, In: What is HTLV-III, Forum on Safe Sex, Nevada State Health Div. AIDS Program, 2009.
Takahashi et al., Cell, 126(4):663-676, 2006.
Takahashi et al., Cell, 131(5):861-72, 2007.
Tamasauskas et al., Blood, 97:3651-3654, 2001.
Wang et al., Nat. Biotechnol., 25(3):317-8, 2007.
Wernig et al., Nature, 448(7151):318-24, 2007
Winkler et al. Science, 279:389-393, 1998.
Woltjen et al., Nature, 458, 766-770, 2009.
Yu et al., Science, 318:1917-1920, 2007.
Yu et al., Science, 324(5928):797-801, 2009.
Zhou et al., Cell Stem Cell, 4 (5), 381-4 2009.
Zwaka and Thomson, Nat. Biotechnol., 21:319-321, 2003.

## Claims

1. An isolated induced pluripotent stem (iPS) cell for use in prevention and therapy of HIV infection, AIDS and/or ARC, wherein said cell has a modified gene structure comprising a mutant form of an HIV cellular entry gene selected from the group consisting of CCR5, CXCR4, CCR3, CCR2B, and CCR1, wherein said mutant form is a null genotype or encodes an inactive protein form.

2. The isolated iPS cell of claim 1, wherein said gene structure is modified by homologous recombination or nuclease targeting.

3. The isolated iPS cell of claim 1 or 2, wherein said CCR5 mutant is a 32 base-pair deletion in the coding region of wild-type CCR5 (CCR5 delta32) and/or wherein said CCR5 mutant is a CCR5m303 mutant.

4. The isolated iPS cell of any one of claims 1 to 3, wherein said isolated iPS cell is homozygous for said CCR5 mutant.

5. An isolated modified hematopoietic cell differentiated from the isolated iPS cell of any one of claims 1 to 4.

6. The isolated iPS cell of claim 5, wherein said isolated hematopoietic cell is a hematopoietic stem cell, a hematopoietic progenitor cell, a T lymphocyte, a B lymphocyte, a mast cell, or a macrophage.

7. An *in vitro* method for making a modified iPS cell for use in prevention and therapy of HIV infection, AIDS and/or ARC, wherein said method comprises:
a) obtaining a somatic cell, wherein said somatic cell is from a subject having or at risk of having an HIV infection or disorder;
b) reprogramming said somatic cell to provide an induced pluripotent stem cell (iPS cell); and
c) modifying said iPS cell to provide a modified iPS cell having a modified gene structure comprising a mutant form of an HIV cellular entry gene selected from the group consisting of CCR5, CXCR4, CCR3, CCR2B, and CCR1.

8. The *in vitro* method of claim 7, further comprising: d) inducing differentiation of said modified iPS cell to provide a modified hematopoietic cell.

9. The *in vitro* method of claim 7 or 8, wherein said modifying comprises homologous recombination or nuclease targeting.

10. The *in vitro* method of any one of claims 7 to 9, wherein said modified gene structure is introduced into said iPS cell by a vector.

11. The *in vitro* method of any one of claims 7 to 10, wherein said modified gene structure comprises a CCR5 null genotype or a CCR5 mutant encoding an inactive form of CCR5.

12. The *in vitro* method of claim 11, wherein said modifying comprises replacing one or two endogenous CCR5 alleles with said CCR5 mutant.

13. The *in vitro* method of claim 11 or 12, wherein said CCR5 mutant is a 32 base-pair deletion in the coding region of wild-type CCR5 (CCR5 delta32) and/or wherein said CCR5 mutant is a CCR5m303 mutant.

14. The *in vitro* method of any one of claims 11 to 13, wherein said modified iPS cell is homozygous for said CCR5 mutants.

15. The *in vitro* method of any one of claims 7 to 14, wherein said subject is human.

## Patentansprüche

1. Isolierte induzierte pluripotente Stammzelle (iPS-Zelle) zur Verwendung bei der Vorbeugung und Therapie einer HIV-Infektion, von AIDS und/oder ARC, wobei die Zelle eine veränderte Genstruktur aufweist, die eine mutierte Form eines Gens für den zellulären Eintritt von HIV ausgewählt aus der Gruppe bestehend aus CCR5, CXCR4, CCR3, CCR2B und CCR1 umfasst, wobei die mutierte Form ein Null-Genotyp ist oder eine inaktive Proteinform codiert.

2. Isolierte iPS-Zelle nach Anspruch 1, wobei die Genstruktur durch homologe Rekombination oder Nuclease-targeting verändert wird.

3. Isolierte iPS-Zelle nach Anspruch 1 oder 2, wobei die CCR5-Mutante eine 32 Basenpaardeletion in dem codierenden Bereich des Wildtyp-CCR5 (CCR5 delta32) ist und/oder wobei die CCR5-Mutante eine CCR5m303-Mutante ist.

4. Isolierte iPS-Zelle nach einem der Ansprüche 1 bis 3, wobei die isolierte iPS-Zelle homozygot bezüglich der CCR5-Mutante ist.

5. Isolierte modifizierte hämatopoetische Zelle, die von der isolierten iPS-Zelle nach einem der Ansprüche 1 bis 4 differenziert ist.

6. Isolierte iPS-Zelle nach Anspruch 5, wobei die isolierte hämatopoetische Zelle eine hämatopoetische Stammzelle, eine hämatopoetische Vorläuferzelle, ein T-Lymphozyt, ein B-Lymphozyt, eine Mastzelle oder eine Makrophage ist.

7. *In vitro*-Verfahren zur Herstellung einer veränderten iPS-Zelle zur Verwendung bei der Vorbeugung und Therapie einer HIV-Infektion, von AIDS und/oder ARC, wobei das Verfahren umfasst:
a) Erhalten einer somatischen Zelle, wobei die somatische Zelle von einem Individuum ist, das eine HIV-Infektion oder -Erkrankung hat oder bei dem das Risiko einer HIV-Infektion oder -Erkrankung besteht;
b) Reprogrammieren der somatischen Zelle, um eine induzierte pluripotente Stammzelle (iPS-Zelle) bereitzustellen; und
c) Verändern der iPS-Zelle, um eine veränderte iPS-Zelle bereitzustellen, die eine veränderte Genstruktur aufweist, die eine mutierte Form eines Gens für den zellulären Eintritt von HIV ausgewählt aus der Gruppe bestehend aus CCR5, CXCR4, CCR3, CCR2B, und CCR1 umfasst.

8. *In vitro*-Verfahren nach Anspruch 7, das zudem umfasst: d) Induzieren der Differenzierung der veränderten iPS-Zelle, um eine veränderte hämatopoetische Zelle bereitzustellen.

9. *In vitro*-Verfahren nach Anspruch 7 oder 8, wobei das Verändern homologe Rekombination oder Nuclease-targeting umfasst.

10. *In vitro*-Verfahren nach einem der Ansprüche 7 bis 9, wobei die veränderte Genstruktur in die iPS-Zelle durch einen Vektor eingeführt wird.

11. *In vitro*-Verfahren nach einem der Ansprüche 7 bis 10, wobei die veränderte Genstruktur einen CCR5-Nullgenotyp oder eine CCR5-Mutante umfasst, die eine inaktive Form von CCR5 kodiert.

12. *In vitro*-Verfahren nach Anspruch 11, wobei das Verändern das Ersetzen von einem oder zwei endogenen CCR5-Allel(en) mit der CCR5-Mutante umfasst.

13. *In vitro*-Verfahren nach Anspruch 11 oder 12, wobei die CCR5-Mutante eine 32 Basenpaardeletion in dem codierenden Bereich des Wildtyp-CCR5 (CCR5 delta32) ist und/oder wobei die CCR5-Mutante eine CCR5m303-Mutante ist.

14. *In vitro*-Verfahren nach einem der Ansprüche 11 bis 13, wobei die veränderte iPS-Zelle homozygot bezüglich der CCR5-Mutanten ist.

15. *In vitro*-Verfahren nach einem der Ansprüche 7 bis 14, wobei das Individuum ein Mensch ist.

## Revendications

1. Cellule souche pluripotente induite (iPS) isolée destinée à être utilisée dans la prévention et le traitement d'une infection par le VIH, du SIDA et/ou de l'ARC, où ladite cellule possède une structure génique modifiée comprenant une forme mutante d'un gène de pénétration cellulaire du VIH sélectionné dans le groupe consistant en CCR5, CXCR4, CCR3, CCR2B et CCR1, où ladite forme mutante est un génotype nul ou code pour une forme protéique inactive.

2. Cellule iPS isolée selon la revendication 1, dans laquelle ladite structure génique est modifiée par recombinaison homologue ou ciblage d'une nucléase.

3. Cellule iPS isolée selon la revendication 1 ou 2, dans laquelle ledit mutant de CCR5 est une délétion de 32 paires de bases dans la région codante du CCR5 de type sauvage (CCR5 delta32) et/ou dans laquelle ledit mutant de CCR5 est un mutant CCR5m303.

4. Cellule iPS isolée selon l'une quelconque des revendications 1 à 3, où ladite cellule iPS isolée est homozygote pour ledit mutant de CCR5.

5. Cellule hématopoïétique modifiée isolée qui est différenciée à partir de la cellule iPS isolée selon l'une quelconque des revendications 1 à 4.

6. Cellule iPS isolée selon la revendication 5, où ladite cellule hématopoïétique isolée est une cellule souche hématopoïétique, une cellule progénitrice hématopoïétique, un lymphocyte T, un lymphocyte B, un mastocyte ou un macrophage.

7. Procédé *in vitro* pour fabriquer une cellule iPS modifiée destinée à être utilisée dans la prévention et le traitement d'une infection par le VIH, du SIDA et/ou de l'ARC, où ledit procédé comprend :
a) l'obtention d'une cellule somatique, où ladite cellule somatique est issue d'un sujet présentant, ou exposé à un risque de présenter, une infection ou un trouble lié(e) au VIH ;
b) la reprogrammation de ladite cellule somatique afin d'obtenir une cellule souche pluripotente induite (cellule iPS) ; et
c) la modification de ladite cellule iPS afin d'obtenir une cellule iPS modifiée ayant une structure génique modifiée comprenant une forme mutante d'un gène de pénétration cellulaire du VIH sélectionné dans le groupe consistant en CCR5, CXCR4, CCR3, CCR2B et CCR1.

8. Procédé *in vitro* selon la revendication 7, comprenant en outre : d) l'induction de la différenciation de ladite cellule iPS modifiée afin d'obtenir une cellule hématopoïétique modifiée.

9. Procédé *in vitro* selon la revendication 7 ou 8, dans lequel ladite modification comprend une recombinaison homologue ou le ciblage d'une nucléase.

10. Procédé *in vitro* selon l'une quelconque des revendications 7 à 9, dans lequel ladite structure génique modifiée est introduite dans ladite cellule iPS par un vecteur.

11. Procédé *in vitro* selon l'une quelconque des revendications 7 à 10, dans lequel ladite structure génique modifiée comprend un génotype nul pour CCR5 ou un mutant de CCR5 codant pour une forme inactive de CCR5.

12. Procédé *in vitro* selon la revendication 11, dans lequel ladite modification comprend le remplacement d'un ou de deux allèles de CCR5 endogènes par ledit mutant de CCR5.

13. Procédé *in vitro* selon la revendication 11 ou 12, dans lequel ledit mutant de CCR5 est une délétion de 32 paires de bases dans la région codante du CCR5 de type sauvage (CCR5 delta32) et/ou dans lequel ledit mutant de CCR5 est un mutant CCR5m303.

14. Procédé *in vitro* selon l'une quelconque des revendications 11 à 13, dans lequel ladite cellule iPS modifiée est homozygote pour lesdits mutants de CCR5.

15. Procédé *in vitro* selon l'une quelconque des revendications 7 à 14, dans lequel ledit sujet est humain.
